(19) Europäisches Patentamt
European Patent Office
Office européen des brevets

(11) EP 2 670 484 B1

(12) **EUROPÄISCHE PATENTSCHRIFT**

(45) Veröffentlichungstag und Bekanntmachung des Hinweises auf die Patenterteilung:
26.10.2016 Patentblatt 2016/43

(21) Anmeldenummer: 10718875.7

(22) Anmeldetag: 23.04.2010

(51) Int Cl.:
*A61N 5/10* (2006.01)

(86) Internationale Anmeldenummer:
PCT/EP2010/002495

(87) Internationale Veröffentlichungsnummer:
WO 2010/121822 (28.10.2010 Gazette 2010/43)

(54) **VERFAHREN ZUR BESTIMMUNG EINER WIRKUNG EINES PARTIKELSTRAHLS IN EINEM MATERIAL**

METHOD FOR DETERMINING AN EFFECT OF A PARTICLE BEAM ON A MATERIAL

PROCÉDÉ DE DÉTERMINATION D'UN EFFET D'UN RAYONNEMENT CORPUSCULAIRE DANS UN MATÉRIAU

(84) Benannte Vertragsstaaten:
AT BE BG CH CY CZ DE DK EE ES FI FR GB GR HR HU IE IS IT LI LT LU LV MC MK MT NL NO PL PT RO SE SI SK SM TR

(30) Priorität: 24.04.2009 DE 102009018545
28.06.2009 DE 102009031772

(43) Veröffentlichungstag der Anmeldung:
11.12.2013 Patentblatt 2013/50

(73) Patentinhaber: GSI Helmholtzzentrum für Schwerionenforschung GmbH
64291 Darmstadt (DE)

(72) Erfinder:
• SCHOLZ, Michael
64390 Erzhausen (DE)
• ELSÄSSER, Thilo
60528 Frankfurt (DE)

(56) Entgegenhaltungen:
• WROE A J ET AL: "Nanodosimetric cluster size distributions of therapeutic proton beams" IEEE TRANSACTIONS ON NUCLEAR SCIENCE IEEE USA, Bd. 53, Nr. 2, April 2006 (2006-04), Seiten 532-538, XP7914520 ISSN: 0018-9499

• SEMENENKO V A ET AL: "Fast Monte Carlo simulation of DNA damage formed by electrons and light ions" PHYSICS IN MEDICINE AND BIOLOGY, TAYLOR AND FRANCIS LTD. LONDON, GB LNKD-DOI:10.1088/0031-9155/51/7/004, Bd. 51, Nr. 7, 7. April 2006 (2006-04-07), Seiten 1693-1706, XP020096207 ISSN: 0031-9155

• SATO TATSUHIKO ET AL: "Biological dose estimation for charged-particle therapy using an improved PHITS code coupled with a microdosimetric kinetic model." RADIATION RESEARCH JAN 2009 LNKD-PUBMED:19138056, Bd. 171, Nr. 1, Januar 2009 (2009-01), Seiten 107-117, XP7914518 ISSN: 0033-7587

• SOLOV'YOV ANDREY V ET AL: "Physics of ion beam cancer therapy: a multiscale approach." PHYSICAL REVIEW. E, STATISTICAL, NONLINEAR, AND SOFT MATTER PHYSICS JAN 2009 LNKD- PUBMED:19257071, Bd. 79, Nr. 1 Pt 1, Januar 2009 (2009-01), Seite 11909, XP7914517 ISSN: 1539-3755

• ELSÄSSER THILO ET AL: "Cluster effects within the local effect model." RADIATION RESEARCH MAR 2007 LNKD-PUBMED:17316069, Bd. 167, Nr. 3, März 2007 (2007-03), Seiten 319-329, XP7914483 ISSN: 0033-7587

- KRÄMER M ET AL: "Treatment planning for heavy-ion radiotherapy: calculation and optimization of biologically effective dose." PHYSICS IN MEDICINE AND BIOLOGY NOV 2000 LNKD- PUBMED:11098906, Bd. 45, Nr. 11, November 2000 (2000-11), Seiten 3319-3330, XP7914482 ISSN: 0031-9155

- SCHOLZ M ET AL: "Computation of cell survival in heavy ion beams for therapy. The model and its approximation." RADIATION AND ENVIRONMENTAL BIOPHYSICS FEB 1997 LNKD- PUBMED:9128899, Bd. 36, Nr. 1, Februar 1997 (1997-02), Seiten 59-66, XP7914481 ISSN: 0301-634X

**Beschreibung**

Die Erfindung betrifft ein Verfahren zur Bestimmung einer Wirkung eines Partikelstrahls in einem zumindest teilweise bestrahlten oder zu bestrahlenden Material, ein Verfahren zur Bestrahlungsplanung für ein Zielvolumen, einen Bestrahlungsplan, eine Strahlmodifikationseinrichtung sowie eine Bestrahlungsvorrichtung.

Die Bestrahlung eines Zielvolumens in einem Bestrahlungsvolumen mit Ionen- oder Partikelstrahlen betrifft die Bestrahlung von Materie, insbesondere anorganischer, organischer und biologischer Materialien und wird in den unterschiedlichen Bereichen der Forschung, der Industrie und der Medizintechnik eingesetzt. Das Zielvolumen umfasst dabei insbesondere den Bereich, in dem zur Modifikation des bestrahlten Materials eine vorgegebene Dosis deponiert werden soll; das Bestrahlungsvolumen umfasst insbesondere auch diejenigen Bereiche des Materials, die von Strahlung durchdrungen werden, um die gewünschte Dosis im Zielvolumen zu erreichen. Unter einem Partikelstrahl oder Ionenstrahl wird insbesondere ein hochenergetischer Strahl aus entweder geladenen Teilchen, beispielsweise Protonen, Kohlenstoffionen oder Ionen anderer Elemente, Pionen oder neutralen Teilchen, beispielsweise Neutronen verstanden. In der nachfolgenden Beschreibung werden die Begriffe Ionenstrahl und Partikelstrahl synonym verwendet. Unter hoher Energie wird insbesondere eine Energie der Partikel im Bereich einiger MeV/amu bis zu einigen GeV/amu (amu: atomare Masseneinheit) verstanden.

Eine zur Durchführung der Bestrahlung geeignete Bestrahlungsvorrichtung weist im Allgemeinen eine den Ionenstrahl erzeugende und formende Beschleunigungseinrichtung auf, wobei der Ionenstrahl zur Bestrahlung über ein Strahltransportsystem in einen Bereich, in dem das Bestrahlungsvolumen angeordnet ist, geführt wird. Ferner umfasst die Bestrahlungsvorrichtung eine Strahlmodifikationseinrichtung, die den Ionenstrahl in seinen Parametern an die Lage und Größe des Zielvolumens anpassen kann.

Das Bestrahlungsvolumen kann beispielsweise ein Detektorsystem sein, das zur Verifikation eines Bestrahlungsfeldes dient. Das Bestrahlungsvolumen umfasst im Allgemeinen ein Bestrahlungsfeld, das ein Feld mit maximaler Ausdehnung in lateraler Richtung ist, im Allgemeinen in x- und y- Richtung und senkrecht zur Richtung des Ionenstrahls liegt. Das Detektorsystem kann dabei aus einem Detektorfeld oder aus einem sogenannten Stack mit mehreren hintereinander angeordneten lateral ausgedehnten Detektorfeldern bestehen. Im Bereich der Dosimetrie werden dazu beispielsweise Filme mit einer photographischen Emulsion verwendet. Weiterhin werden Kernspurdetektoren zur Messung der Fluenzverteilung in dem Bestrahlungsfeld eingesetzt. Im Bereich medizinischer Anwendungen wird die Bestrahlung von biologischem Gewebe zum Studium der Wirkung von Partikelstrahlung genutzt, um die Wirkung der Strahlenexposition kosmischer Strahlung im Weltraum abschätzen zu können. Schließlich kann das Bestrahlungsvolumen auch das Volumen eines Tumors in einem Patienten sein. Ionenstrahlen werden hierbei verwendet, um Tumorgewebe im Zielvolumen zu zerstören.

Bei der Tumortherapie erlauben die besonderen Eigenschaften von Ionenstrahlen, das Tumorgewebe bei minimaler Schädigung des umliegenden gesunden Gewebes zu zerstören. Dies hängt mit der günstigen Tiefendosisverteilung von Ionenstrahlen zusammen. Beim Eindringen von hochenergetischen Ionenstrahlen in das Material deponieren diese zunächst wenig Energie. Mit zunehmender Tiefe steigt die Energiedeposition an, erreicht im Bereich einer als Bragg-Peak bezeichneten Verteilungskurve ihr Maximum und fällt danach steil ab. Dadurch lässt sich auch bei tiefer liegenden Tumoren im Tumorgewebe mehr Energie deponieren als im umliegenden gesunden Gewebe.

Ionenstrahlen zeigen dabei im Bestrahlungsvolumen eine Wirkung, die von der Art des zu bestrahlenden Materials und von den Parametern des Ionenstrahls abhängig ist. Im Allgemeinen zeigen Ionenstrahlen eine andere Wirkung als Photonenstrahlung. Das bedeutet, dass mit Ionenstrahlen eine andere Dosis deponiert werden muss, als mit Photonenstrahlen, um eine vorgegebene Wirkung bzw. einen vorgegebenen Bestrahlungseffekt zu erzielen. Als effektive Dosis wird dabei diejenige Photonendosis $D_\gamma$ bezeichnet, die zum selben Bestrahlungseffekt wie die Ionendosis $D_I$ führen würde. Die veränderte Wirkung von Ionenstrahlen wird sowohl für anorganisches, als auch für organisches und für biologisches Material beobachtet. In anorganischen Materialien wird eher eine geringere Wirkung von Ionenstrahlen im Vergleich zu Photonenstrahlen beobachtet. Bei der Bestrahlung von biologischem Material mit Ionen hingegen wird in der Regel eine höhere Wirkung und somit ein größerer Effekt verglichen mit Photonenbestrahlung beobachtet.

Vor der eigentlichen Bestrahlung wird im Allgemeinen ein Bestrahlungsplan zur Durchführung der Bestrahlung des Zielvolumens, beispielsweise eines Teilbereiches in einem Phantom oder ein Tumor, erstellt. Im Falle der Bestrahlung mit Ionenstrahlen soll dieser Bestrahlungsplan möglichst die Wirkung der Ionenstrahlen berücksichtigen.

Es sind unterschiedliche Verfahren zur Erstellung eines Bestrahlungsplanes bekannt. Beispielsweise wird in der Veröffentlichung (Krämer und Scholz 2000, Physics in Medicine and Biology, Vol. 45, Seiten 3319-3330) ein Verfahren zur Erstellung eines Bestrahlungsplanes beschrieben.

Die Wirkung der Ionenstrahlen in dem Material hängt in komplexer Weise von der Ionensorte, der Ionenenergie, von der Bestrahlungsdosis, vom bestrahlten Material, und vom jeweils betrachteten Effekt ab. Die experimentelle Bestimmung dieser vielfältigen Abhängigkeiten mit der Genauigkeit, die für die Bestrahlungsplanung notwendig ist, ist praktisch undurchführbar. Deshalb stellen Modelle, die eine Vorhersage der veränderten Wirksamkeit erlauben, ein wichtiges Werkzeug für die Realisierung der Bestrahlungsplanung dar. Diese Modelle beruhen in der Regel auf Vereinfachungen und Näherungen, da die zugrunde liegenden Mechanismen der Schädigung von anorganischem, organischem und biologischem Material noch nicht hinreichend genau quantitativ aufgeklärt sind. Dementsprechend ist im Allgemeinen der Anwendungsbereich der Modelle auch limitiert.

Ein Beispiel eines derartigen Modells wird in der Veröffentlichung (Scholz et al, Radiation Environmental Biophysics, Vol. 36, S. 59-66 (1997)) beschrieben. Das Modell wird als LEM bezeichnet, wobei der Begriff LEM die Abkürzung für "local effect model" ist.

Die bisher bekannten Modelle können keine Aussagen für die Bestrahlungsplanung über den gesamten Bereich von leichten bis schweren Ionen mit einer ausreichenden Genauigkeit liefern.

Deshalb besteht Bedarf, die Wirkung von Partikelstrahlen, insbesondere Ionenstrahlen mit Hilfe eines Modells, über einen weiten Massenbereich, insbesondere von Protonen bis Neon-Ionen, zuverlässig mit der erforderlichen Genauigkeit zu beschreiben und vorherzusagen.

Die Aufgabe der vorliegenden Erfindung ist es, ein gegenüber dem Stand der Technik verbessertes Verfahren zur Bestimmung der Wirkung eines Partikelstrahls auf ein zu bestrahlendes oder bestrahltes Material zu schaffen. Weiterhin soll ein Verfahren zur Bestrahlungsplanung für ein Zielvolumen und zur Bestrahlung eines Zielvolumens geschaffen werden. Ferner soll eine verbesserte Bestrahlungsvorrichtung geschaffen werden.

Die jeweilige Aufgabe wird durch die Merkmale der unabhängigen Ansprüche gelöst. Weiterbildungen der vorgeschlagenen Verfahren und der vorgeschlagenen Vorrichtungen ergeben sich aus den abhängigen Ansprüchen.

In dem vorgeschlagenen Verfahren wird eine Wirkung eines Partikelstrahls in einem zumindest teilweise bestrahlten oder zu bestrahlenden Material ermittelt, wobei aus zumindest einem Parameter, der den Partikelstrahl charakterisiert, und aus zumindest einem Parameter, der die Materialeigenschaften charakterisiert, die Wirkung des Partikelstrahls in dem Material zumindest teilweise auf Basis einer mikroskopischen Schadenskorrelation bestimmt wird.

Unter der mikroskopischen Schadenskorrelation wird insbesondere eine räumliche Zusammenwirkung der im Material entstandenen Schäden oder Schadensereignisse bevorzugt auf einer Längenskala verstanden. Die Größe der verwendeten Längenskala richtet sich hierbei bevorzugt nach einer für das Material und der entstandenen Schäden und deren räumliche Zusammenwirkung sinnvollen Größenordnung. Diese Längenskala kann sich je nach bestrahltem oder zu bestrahlenden Material unterscheiden, insbesondere unterschiedlich groß sein. Die Schadenskorrelation der Schäden für ein anorganisches Material kann sich hierbei von den Schäden in einem biologischen Material unterscheiden.

Die Wirkung des Partikelstrahls in dem Material wird hierbei typischerweise von den Merkmalen des Partikelstrahls, wie beispielsweise der Energie, der Ionensorte und/oder dem linearen Energietransfers des Partikelstrahls im Material (linear energy transfer: LET) beeinflußt. Mit anderen Worten ist die Wirkung des Partikelstrahls unter anderem abhängig von der Energie des Partikelstrahls, der Masse und dem Ladungszustand des einzelnen Partikels in dem Partikelstrahl und der in das Material transferierten Energie des Partikelstrahls. Eine typische Energie für einen Partikelstrahl liegt im Bereich von einigen hundert keV GeV/amu bis einige zehn GeV/amu, wobei der Begriff amu eine atomare Masseneinheit bezeichnet.

Die Materialeigenschaft beschreibt im Allgemeinen eine Eigenschaft des Materials und betrifft eine Empfindlichkeit des Materials gegenüber einem Energieeintrag, der eine Energiedeposition und somit eine in dem Material deponierte Dosis betrifft. Durch den Energieeintrag kann ein Schadensereignis induziert werden, sodass die Empfindlichkeit die Energiedeposition beschreibt, die notwendig ist, um ein Schadensereignis zu induzieren. Dies kann die Empfindlichkeit eines Polymermaterials sein, die Empfindlichkeit eines Röntgenfilms oder die Empfindlichkeit von biologischem Gewebe. Diese Empfindlichkeit des Materials wird typischerweise für die Bestrahlung eines Materials mit Photonenstrahlung ermittelt und gemessen und ist somit eine Materialeigenschaft.

In einer Weiterbildung des Verfahrens kann die mikroskopische Schadenskorrelation im Submikrometerbereich erfolgen. Die Längenskala liegt insbesondere für den Fall, dass das Material ein biologisches Material ist, im Submikrometerbereich. Der Submikrometerbereich umfasst einen Längenbereich von einigen hundert Nanometern, insbesondere größer als ca. 100 nm. Bevorzugt umfasst die Längenskala einen Bereich zwischen etwa 400 und 500 nm, besonders bevorzugt 440 nm. Grundsätzlich kann es sich bei den Schäden oder Schadensereignissen um Veränderungen im Material oder von Bestandteilen des Materials handeln, die durch den Energieverlust im Material entstanden sind. Diese Veränderung kann eine chemische Veränderung einer chemischen Verbindung, beispielsweise der Zerfall von anorganischen oder organischen Molekülen, der Bruch in einer Polymerkette, die Abspaltung von Seitenketten oder Seitengruppen in einem Polymermaterial und/oder ein oder mehrere Einzelstrangbrüche oder Doppelstrangbrüche der DNA in einem biologischen Material sein. Mit anderen Worten, können die durch den Partikelstrahl induzierten Schäden oder Schadensereignisse in eine räumliche Beziehung zueinander gesetzt werden, wobei bevorzugt alle entstandenen oder zu erwartenden Schäden oder Schadensereignisse auf einer Längenskala von ungefähr 100 nm oder größer, aber kleiner als ungefähr 1000 nm betrachtet werden. In biologischem Material, beispielsweise in Zellen oder aus Zellen zusammengesetzten Geweben können insbesondere die auftretenden oder zu erwartenden DNA-Doppelstrangbrüche in einem Abstand von ungefähr 440 nm betrachtet werden und als charakteristische Größe für die Wirkung des Partikelstrahls im Material herangezogen werden. Die Auflösung der Längenskala kann hierbei bevorzugt kleiner sein als die Längenskala, insbesondere im Bereich von ungefähr 10 nm liegen.

Die mikroskopische Schadenskorrelation kann unter Verwendung einer räumlichen mikroskopischen Schadensverteilung ermittelt werden. Außerdem kann die räumliche mikroskopische Schadensverteilung zumindest teilweise aus einer mikroskopischen Dosisverteilung ermittelt werden. Somit kann die mikroskopische Schadensverteilung ermittelt werden, die durch den Partikelstrahl erzeugt wird, wobei die Wahrscheinlichkeit einer lokalen Schadensinduzierung zumindest teilweise aus einer ersten Photonendosiseffektkurve abgeleitet wird, die die Anzahl von Schäden pro Dosiseinheit beschreibt. Ferner kann gemäß einer Weiterbildung des Verfahren bei der Bestimmung der mikroskopischen Schadenskorrelation ein Erwartungswert für eine Anzahl korrelierter Schäden in einem geeignet gewählten Teilvolumen eines sensitiven Volumens zumindest teilweise aus der räumlichen, mikroskopischen Schadensverteilung bestimmt werden, insbesondere das Verhältnis aus Anzahl korrelierter Schäden und der durch den Partikelstrahl deponierten Dosis und damit der insgesamt induzierten isolierten und korrelierten Schäden. Es kann ferner eine Photonendosis ermittelt werden, die zum Erreichen derselben Ausbeute korrelierter Schäden entsprechend dem für den Partikelstrahl erwarteten Wert der Anzahl korrelierter Schäden erforderlich gewesen wäre. Hierbei kann die Ausbeute korrelierter Schäden das Verhältnis aus der Anzahl korrelierter Schäden und der durch die Bestrahlung deponierten Dosis beschreiben. Alternativ kann die Ausbeute korrelierter Schäden auch das Verhältnis aus der Anzahl korrelierter Schäden und der Anzahl isolierter Schäden beschreiben. Gemäß einer Ausgestaltung des Verfahrens kann ein zu dieser Photonendosis gehöriger Effekt zumindest teilweise aus einer zweiten Photonendosiseffektkurve bestimmt werden. Die Wirkung des Partikelstrahls kann zumindest teilweise aus einer Skalierung des zur Photonendosis gehörigen Effekts entsprechend dem Verhältnis der Photonendosis und einer im sensiblen Volumen durch den Partikelstrahl deponierten Dosis bestimmt werden. Bevorzugt kann die Wirkung des Partikelstrahls zumindest teilweise aus einer Skalierung des zur Photonendosis gehörigen Effektes entsprechend der Anzahl korrelierter Schäden und der durch Ionenbestrahlung induzierten Anzahl korrelierter Schäden bestimmt werden.

Die in den Verfahrensschritten zur Bestimmung der Wirkung eines Partikelstrahls auf ein Material verwendeten Begriffe sollen im Folgenden näher definiert werden.

Die räumliche, mikroskopische Schadensverteilung (rSv) kann im Allgemeinen durch eine räumliche Verteilung der Schäden oder Schadensereignisse bestimmt sein. Die räumliche, mikroskopische Schadensverteilung kann zumindest teilweise aus der mikroskopischen Dosisverteilung (mDv) ermittelt werden.

Die mikroskopische Dosisverteilung, insbesondere die lokale Dosisverteilung, kann zumindest unter teilweiser Verwendung der radialen Dosisverteilung um eine einzelne Ionenspur bestimmt werden. Die radiale Dosisverteilung beschreibt den Erwartungswert der lokalen Energiedeposition als Funktion des Abstandes von der Trajektorie der Ionenspur. Der Vorteil der Verwendung der radialen Dosisverteilung liegt darin, dass damit direkt auf die Effekte nach Photonenbestrahlung Bezug genommen werden kann. Die radiale Dosisverteilung kann beispielsweise durch Monte Carlo Simulationen berechnet werden. Eine weitere Möglichkeit kann sich durch eine analytische Dosisbeschreibung im Sinne einer amorphen Bahnstruktur ergeben.

Die Photonendosiseffektkurven an sich sind bekannt und werden in der Regel experimentell ermittelt. Beispielsweise kann die mittlere Anzahl an Schäden häufig durch einen linear-quadratischen Zusammenhang beschrieben werden:

$$N_{Schaden} = \gamma D_X + \delta D_X \quad (1)$$

$D_X$ ist hierbei die Röntgen- oder Photonendosis und $\gamma$ und $\delta$ sind materialspezifische Konstanten, wie etwa Parameter zur Beschreibung der Anzahl von Polymerisations-Ereignissen pro Dosiseinheit in einem Monomer-Kristall eines GafChromic-Films oder Parameter zur Anzahl von Doppelstrangbrüchen pro Dosiseinheit.

Die Wirkung von Photonenstrahlen ist dadurch charakterisiert, dass die räumliche Verteilung der entstehenden Schäden im Allgemeinen auf Grund der physikalischen Eigenschaften der Energiedeposition von Photonen gleichförmig stochastisch verteilt ist. Wenn das sensitive Volumen in Teilvolumina, die auch als Parzellen bezeichnet werden, aufgeteilt wird, kann man davon ausgehen, dass jede Parzelle die gleiche Schadensdichteverteilung aufweist. Dies bedeutet, dass eine gleichmäßige Schadensverteilung in jeder Parzelle vorliegt.

Für ein mit Ionen bestrahltes sensitives Volumen hingegen, das in Parzellen aufgeteilt ist, liegt aufgrund der extrem lokalisierten Energiedeposition von Ionenstrahlen, eine heterogene Schadensverteilung vor. Wird das sensitive Volumen in Parzellen mit einer Größe von kleiner oder kleiner/gleich $1{\times}1{\times}1$ nm$^3$ ($\leq 1{\times}1{\times}1$ nm$^3$) aufgeteilt und in jeder Parzelle die mikroskopische Dosis, insbesondere lokale mikroskopische Dosisverteilung bestimmt, kann somit eine mikroskopische, räumliche Schadensverteilung rSv ermittelt werden.

Der Erwartungswert für die Anzahl korrelierter Schäden (AkS) in einem geeignet gewählten Teilvolumen eines sensitiven Volumens kann zumindest teilweise aus der räumlichen, mikroskopischen Schadensverteilung genauer bestimmt werden. Hierbei ist mit dem Begriff "korrelierter Schaden", ein Schaden gemeint, der durch das räumliche Zusammenwirken von Einzelschäden entstehen kann. Die räumliche Korrelation kann dabei durch eine Analyse der Abstände der Einzelschäden definiert werden. Im Falle von biologischem Material kann ein korrelierter Schaden zum Beispiel durch die Kombination von zwei Doppelstrangbrüchen gegeben sein, die zu einem Schaden führen, der für das biologische Material schlechter zu reparieren ist. Der Vorteil der Verwendung korrelierter Schäden liegt darin begründet, dass damit insbesondere die nicht-lineare Reaktion von Materialien oder Zellen auf Bestrahlung besser berücksichtigt werden kann.

Eine Photonendosis (PD1) kann ermittelt werden, die zum Erreichen derselben Ausbeute korrelierter Schäden, d.h. derselben Anzahl korrelierter Schäden (AkS) bezogen auf die Gesamtzahl der Einzelschäden oder die Anzahl isolierter Schäden (AiS), wie nach Ionenbestrahlung erforderlich gewesen wäre. Das bedeutet, dass zu einer makroskopischen Photonendosis innerhalb des Bestrahlungsfeldes eine räumliche Verteilung der Schäden, deren Art und deren Anzahl gemäß der Photonen-Dosiseffektkurve PEK1, ermittelt wird, aus der der Erwartungswert der Anzahl der korrelierten Schäden bestimmt werden kann. Insbesondere ergibt sich somit einer Photonendosis PD1, die zur gleichen Ausbeute korrelierter Schäden wie nach Ionenstrahlung führt. Beispielsweise kann man für den Erwartungswert der Anzahl von Doppelstrangbruchpaaren innerhalb eines vorgegebenen Abstands annehmen, dass dieser quadratisch von der makroskopischen Photonendosis abhängt. Insbesondere ermöglicht dies eine schnelle Bestimmung von PD1 basierend auf der Simulation der Ausbeute von korrelierten Schäden bei einer bestimmten Photonendosis. Dazu wird angenommen, dass bei genügend klein gewählten Parzellen und bei gleicher lokaler Dosis mit Photonen- und mit Ionenbestrahlung ein gleicher Schaden induziert wird. Dies kann auch damit begründet werden, dass bei einem genügend kleinem Parzellen-Volumen auch im Falle der Ionenstrahlen der Erwartungswert der Energiedeposition in diesem Volumen als homogen verteilt angenommen werden kann und damit direkt mit der Wirkung der Bestrahlung von Photonenstrahlen verglichen werden kann. Hierbei ist vorteilhaft, dass die Anzahl der Schäden, die aus Photonenbestrahlung bekannt sind, auf ein mit Ionen bestrahltes Material in dieser Dimension übertragen werden können.

Aus einer zweiten Photonendosiseffektkurve für den beobachtbaren Effekt (PEK2) kann zumindest teilweise ein zu der Photonendosis (PD1) gehöriger Effekt (E1) bestimmt werden. Im Gegensatz zur Photonen-Dosiseffektkurve (PEK1), die den lokalen, mikroskopischen bzw. molekularen Schaden beschreibt, repräsentiert die Photonen-Dosiseffektkurve (PEK2) den makroskopisch beobachtbaren Effekt. Im Falle von Zellen kann PEK2 z.B. die Inaktivierung von Zellen beschreiben, beispielsweise charakterisiert durch die linear-quadratischen Parameter $\alpha_x$ und $\beta_x$, die für viele Zell- und Gewebearten bekannt sind. Im Falle von Geweben bzw. Organen kann PEK2 die Wahrscheinlichkeit von Gewebeschädigungen bzw. Organversagen beschreiben. Ein Vorteil liegt ähnlich wie bei PEK1 darin, dass durch Bezugnahme auf die experimentellen Photonen-Daten, mit PEK2 eine hohe Genauigkeit der ermittelten biologischen Effekte für Ionenbestrahlung erreicht werden kann. Ferner können mit diesem Verfahren im Vergleich zu anderen Verfahren die Rechenzeiten drastisch reduziert werden.

Der beobachtbare Effekt (E2) kann zumindest teilweise aus der Skalierung des biologisch relevanten Effekts (E1) entsprechend dem Verhältnis aus der Photonendosis (PD1) und der im sensitiven Volumen deponierten Ionendosis (ID) bestimmt werden. Insbesondere kann der beobachtbare Effekt (E2) wie folgt berechnet werden:

$$E2 = E1 \cdot \frac{AkS_2}{AkS_1} \quad (2)$$

wobei beispielsweise angenommen werden kann, dass E2 durch ein einzelnes Ion entstanden ist mit zugehöriger Ionendosisdeposition ID und zugehöriger Photonendosis PD1 Ebenfalls kann beispielsweise angenommen werden, dass E2 durch ein einzelnes Ion entstanden ist und AkS1 bzw. AkS2 die Anzahl korrelierter Schäden durch die Photonendosis PD1 bzw. die Ionendosis ID beschreiben.

Alternativ kann die Wirkung des Partikelstrahls auch direkt aus der Anzahl der isolierten und korrelierten Schäden bestimmt werden, sofern der Effekt dieser Schadenstypen aus der Photonendosiseffektkurve abgeleitet werden kann:

$$E2 = AiS2 \cdot \varepsilon_i + AkS2 \cdot \varepsilon_k \quad (3)$$

Die effektive Dosis kann zumindest teilweise aus der dem beobachtbaren Effekt (E2) zugehörigen Photonendosis (PD2) bestimmt werden. Durch geeignete Optimierungsverfahren kann damit die Ionendosis für jeden Punkt eines Bestrahlungsfeldes so gewählt werden, dass damit ein vorgegebener Effekt in jedem Punkt des Bestrahlungsvolumens erzielt werden kann. Im Falle eines gemischten Strahlenfeldes kann beispielsweise PD2 auch aus der Betrachtung des Strahlenfeldes an jedem Punkt des Bestrahlungsfeldes gemäß der im Stand der Technik bekannten Methoden bestimmt werden. Dabei kann insbesondere die so genannte intrinsische RBW von einzelnen Ionen benutzt werden, wobei sich die intrinsische RBW wie folgt ermitteln lässt:

$$RBW_{\text{int}} = \frac{\alpha_{\text{int}}}{\alpha_X} = \frac{E2_{Single}}{ID_{\sin gle} \cdot \alpha_X} \, , \, (4)$$

wobei $\alpha_{\text{int}}$*$ID_{single}$ dem biologischen Effekt $E2_{single}$ eines einzelnen Ions mit Dosisdeposition $ID_{single}$ entspricht. Gemäß der Veröffentlichung (Krämer und Scholz, Physics in Medicine and Biology, Vol. 51, S 1959- 1970, 2006.) lässt sich $\beta_{\text{int}}$ aus $\alpha_{\text{int}}$ gewinnen und aus $\alpha_{\text{int}}$ und $\beta_{\text{int}}$ für das gesamte gemischte Strahlenfeld letztlich der Effekt E2. Dabei kann der Effekt nach Ionenbestrahlung beispielsweise mittels der linear-quadratischen Parameter $\alpha_{\text{ion}}$ und $\beta_{\text{ion}}$ beschrieben werden.

Unter Bestimmung und/oder Ermittelung kann im vorstehend gesagten insbesondere verstanden werden, dass die jeweiligen Größen in einem komplexen technischen Verfahren in einer Recheneinheit berechnet werden. Der Vorteil des Verfahrens zur Bestimmung der Wirkung eines Partikelstrahls in einem Material oder auf ein Material ist darin zu sehen, dass es eine direkte Übertragung der Erfahrungen mit konventioneller Bestrahlung auf die Bestrahlung mit Teilchenstrahlen erlaubt. Ein weiterer Vorteil des vorgeschlagenen Verfahrens besteht darin, dass die RBW$_{int}$-Werte und/oder oder lineare, quadratische Koeffizienten im Voraus unabhängig vom aktuellen Strahlenfeld für einzelne Ionen von Protonen bis Neon für Energien von 0.1 MeV/u bis 1 GeV/u vorherberechnet und in Form von Tabellen gespeichert werden können, bevor E2 für das gemischte Strahlenfeld berechnet wird. Dies kann zu einer weiteren erheblichen Ersparnis an Rechenzeit führen.

Die weiteren Vorteile und Eigenschaften dieser Weiterbildungen ergeben sich in analoger Weise aus der nachfolgenden Beschreibung des Verfahrens zur Bestrahlungsplanung und/oder des Verfahrens zur Bestrahlung eines Zielvolumens, welches nicht Teil der Erfindung ist, sowie der Bestrahlungsvorrichtung sowie deren Weiterbildungen.

Das vorgeschlagene Verfahren zur Bestrahlungsplanung für ein Zielvolumen mit einem Partikelstrahl weist die Schritte auf: -Vorgeben eines Zielvolumens in dem Bestrahlungsvolumen, - Ermitteln einer Fluenz- und/oder Energieverteilung des Partikelstrahls innerhalb eines das Zielvolumen aufweisende Bestrahlungsvolumen; -Ermitteln einer aus der Fluenz- und/oder Energieverteilung resultierenden effektiven Dosisverteilung, wobei Daten verwendet werden, der Wirkung im Material des Bestrahlungsvolumens zumindest teilweise auf der Basis einer mikroskopischen Schadenskorrelation bestimmen. Hierbei kann der Verfahrensschritt des Vorgebens eines Zielvolumens in einem Bestrahlungsvolumen der Bestimmung einer Fluenz- und/oder Energieverteilung vorangehen.

Im Verfahren wird typischerweise für eine Fluenz- und/oder Energieverteilung eines Partikelstrahls die im Zielvolumen erzeugte Wirkung, bestimmt, wobei die die effektive Dosisverteilung bevorzugt ermittelt wird. Die Bestimmung der Wirkung beruht bevorzugt auf der Basis der mikroskopischen Schadenskorrelation. Dies bezeichnet eine Zusammenwirkung der Schadensereignisse auf einer Submikrometerskala, die bevorzugt größer als etwa 100 nm ist.

Hierbei ist das Zielvolumen in der Regel ein zu bestrahlendes Volumen in einem Gegenstand. Der Gegenstand kann ein abgegrenztes Volumen in einem zu bestrahlenden Material, beispielsweise ein Detektorsystem, ein Röntgenfilm, ein Phantom zur Simulation einer Bestrahlungssituation oder ein Mensch sein. Das Bestrahlungsvolumen kann das Zielvolumen, das vor dem Zielvolumen angeordnete, als auch das nach dem Zielvolumen -in Richtung der Teilchenstrahlen gesehen- angeordnete Material sein. Hierbei liegt das Material des Zielvolumens, beispielsweise das zu modifizierende Material eines Gegenstandes oder der zu zerstörende Tumor, in der Regel im Bereich des Bragg-Peaks des Partikelstrahls.

Die Fluenzverteilung, die üblicherweise die Anzahl der Ionen- oder Partikeldurchgänge pro Flächeneinheit (Ionen/cm$^2$) beschreibt sowie die zugehörige Energieverteilung, wird hierbei üblicherweise an mindestens einem Punkt des Zielvolumens, vorzugsweise aber in einer dreidimensionalen (3D) Anordnung oder Matrix von Punkten des Zielvolumens ermittelt. Die Fluenzverteilung kann neben der Information über die primären Ionenstrahlen auch Informationen über die durch Kernreaktionen erzeugten Sekundärteilchen enthalten.

Aus der Fluenz- und Energieverteilung kann eine resultierende makroskopische, physikalische Dosisverteilung ermittelt werden. Unter der makroskopischen physikalischen Dosisverteilung wird im Folgenden die Dosisverteilung verstanden, die sich aus dem Erwartungswert der Dosisdeposition der Teilchenstrahlen in Volumina von typischerweise einigen Kubikmillimetern oder größer ergibt. Diese Dosisverteilung basiert auf der Kenntnis der Anzahl der Teilchen und deren Energiedeposition an jedem Punkt $x_i$, $y_i$, $z_i$ des Zielvolumens.

Zur Beschreibung des zu erwartenden Bestrahlungseffektes ist jedoch im Falle von Ionen- oder Partikelstrahlen die makroskopische Dosisverteilung alleine nicht ausreichend. Die Strahlenwirkung wird maßgeblich auch durch die veränderte Wirksamkeit von Ionenstrahlen im Vergleich zu konventionellen Photonenstrahlen bestimmt.

Das Ziel der Bestrahlungsplanung ist es in der Regel, die effektive Dosis mit einer Genauigkeit von ca. 5-10% bestimmen zu können. Als effektive Dosis wird hierbei diejenige Dosis bezeichnet, die mit Photonenstrahlen deponiert werden müsste, um denselben Effekt wie mit Ionenstrahlen zu erreichen. Ausgangsbasis zur Berechnung des Bestrahlungsplans ist deshalb der erzielte, beobachtbare, beziehungsweise gewünschte Wirkung oder der gewünschte Effekt im Material des Zielvolumens. Hierbei werden Parameter der zu applizierenden Ionenstrahlen, wie beispielsweise Ionenstrahlsorte, Ionenstrahlenergien und/oder üblicherweise die veränderte Wirksamkeit im Vergleich zu Photonenstrahlen herangezogen und daraus die zu applizierende Dosis bezogen auf das Zielvolumen ermittelt bzw. berechnet.

Das LEM gemäß dem Stand der Technik erlaubt es, die Wirksamkeit von Ionenstrahlen aus der Kenntnis der physikalischen Eigenschaften von Ionenstrahlen sowie der Kenntnis der Reaktion von Materialien auf Photonenstrahlung abzuleiten. Aufgrund der verwendeten Vereinfachungen und Näherungen ist die Genauigkeit des Modells in der Regel aber nur für Anwendungen bei schwereren Ionen wie z.B. Kohlenstoff genügend genau für die Bestrahlungsplanung. Ausgehend von der ursprünglichen Implementierung (LEM I) konnte zwar die Genauigkeit der Rechnung durch Weiterentwicklungen verbessert werden (LEM II: Elsässer und Scholz 2007, Radiation Research Vol. 167, 319-329; LEM III: Elsässer et al. 2008, International Journal of Radiation Oncology Biology Physics, Vol. 71, 866-872); das Modell bietet aber noch keine ausreichende Genauigkeit für eine generelle Anwendung über einen weiten Massenbereich der Ionen (Protonen - Neonionen) sowie Energiebereich (1 MeV/u - 1 GeV/u).

Im Gegensatz zu den bisher verwendeten Verfahren in den Modellen LEM I bis LEM III wird bei dem vorgeschlagenen Verfahren die mikroskopische räumliche Schadenskorrelation zur Bestimmung der effektiven Dosis verwendet. Für die Bestrahlung von nicht biologischem, beispielsweise anorganischem Material sind zur Bestimmung der veränderten Wirksamkeit und damit der effektiven Dosis insbesondere Materialkonstanten, wie beispielsweise die Empfindlichkeit von Silberbromid-Kristallen in Röntgenfilmen oder die Färbung radiochromer Farbstoffe nach Bestrahlung maßgeblich. Im Falle der Bestrahlung von biologischem Material, beispielsweise von Tumorzellen, stellen Schädigungen der im Zellkern enthaltenen DNA üblicherweise die maßgebliche Ursache für die beobachtbaren Strahlenwirkungen dar. Zur Beschreibung der effektiven Dosis wird hier häufig die relative biologische Wirksamkeit (RBW) der Ionen verwendet, die durch das Verhältnis der Ionendosen definiert ist, die zur Erzielung desselben Effektes mit Photonenstrahlen bzw. Ionenstrahlen notwendig sind.

$$RBW = \frac{D_{Photon}}{D_{Ion}}\bigg|_{Isoeffekt} \qquad (5)$$

Hierbei bezeichnet der Begriff Isoeffekt der gleiche Effekt für $D_{Photon}$ und $D_{Ion}$. Dies bedeutet, dass zunächst die Schadensart bzw. der beobachtbare Effekt definiert und als Parameter verwendet werden muss.

In einer Ausgestaltung des Verfahrens zur Bestrahlungsplanung kann für verschiedene Parameter, die den Partikelstrahl charakterisieren und/oder verschiedene Eigenschaften des Materials jeweils der Effekt des Partikelstrahls auf das zu bestrahlende Material auf Basis der mikroskopischen Schadenskorrelation bestimmt werden. Die jeweils für die verschiedenen Parameter des Partikelstrahls und für die verschiedenen Materialeigenschaften ermittelten Effekte bzw. die die Effekte charakterisierenden Daten und Werte können in einer Speichereinheit hinterlegt werden.

Auf diese Weise können für verschiedene Eigenschaften des Partikelstrahls, z.B. für Ionen von Protonen bis Neon und/oder für Energien von 0.1 MeV/u bis 1 GeV/u Effekte vorherberechnet werden und in einer Speichereinheit, z.B. einem Datensatz und/oder einer Datentabelle hinterlegt werden. Die hinterlegten Effekte können dann in einem Verfahren zur Bestrahlungsplanung verwendet werden.

Um die zur Erzielung des vorgegebenen Effektes erforderliche Dosis ermitteln zu können, können die entsprechenden Materialkonstanten bzw. die zur Beschreibung der relativen biologischen Wirksamkeit benötigten Daten, die linearen und/oder quadratischen Koeffizienten der Dosiseffektkurven oder andere Koeffizienten vorzugsweise in Form eines Datensatzes oder einer die Daten enthaltenden Tabelle verwendet werden.

Beispiele für solche Datensätze sind Tabellen mit Parametern für bestimmte Ionenenergien und Ionensorten, die die Ermittlung der relevanten RBW-Werte erlauben. Der diese Tabellen enthaltende Datensatz kann als RBW-Datensatz bezeichnet werden und kann in Form einer Parameter-Tabelle, die zur Ansteuerung einer Bestrahlungsvorrichtung verwendet wird, vorliegen. Es ist aber auch denkbar, das Verfahren zur Bestimmung des RBW-Datensatzes in eine Steuerungseinrichtung einer Bestrahlungsvorrichtung direkt zu implementieren. Der Datensatz kann als Parameterfeld in einer SteuerEinrichtung zur Steuerung einer Bestrahlungsvorrichtung, beispielsweise eines Beschleunigers, vorliegen.

Aus der Fluenzverteilung kann darüber hinaus die lokale Dosisverteilung an jeder Stelle in einem Volumen von typischerweise 10 x 10 x 10 $\mu$m$^3$, mit einer Auflösung von typischerweise <1 nm$^3$, d.h. im Subnanometer-Bereich, in einem Bestrahlungsfeld bestimmt werden. Die lokale Dosisverteilung kann ebenfalls die spektrale Verteilung der Teilchen berücksichtigen. Die lokale Dosisverteilung ist demnach eine mikroskopische Dosisverteilung im Gegensatz zu der vorstehend definierten makroskopischen Dosisverteilung.

Die Ermittlung der Wirkung des Partikelstrahls auf ein Material bzw. eine effektive Dosisverteilung beispielsweise im Rahmen einer Bestrahlungsplanung kann auf der Berücksichtigung einer lokalen Schadenskorrelation beruhen, die mit Hilfe der lokalen, mikroskopischen Dosisverteilung bestimmt werden kann. Die Wahrscheinlichkeit für die Erzeugung korrelierter Schäden, insbesondere räumlich korrelierter Schäden bzw. Schadensereignisse, hängt unter anderem von der Anzahl der Schäden in einem Teilvolumen eines geeignet gewählten sensitiven Volumens ab. Im Falle von Filmen kann das sensitive Volumen durch das Volumen eines Silberbromid-Korns definiert sein; im Falle von Zellen kann dies der Zellkern sein. Als korrelierte Schäden werden dabei solche Schäden bezeichnet, die durch Zusammenwirken einzelner Schäden innerhalb eines geeignet gewählten Abstandes entstehen. Als einzelne Schäden können beispielsweise Polymerisations-Ereignisse in einem Monomer-Kristall eines GafChromic-Films oder DNA-Schäden in einem Zellkern eines biologischen Gewebes angesehen werden. Die Wahrscheinlichkeit zur Erzeugung korrelierter Schäden kann demnach zum Beispiel aus einer Abstands-Analyse der Schäden ermittelt werden. Ein einfaches Beispiel für die Ermittlung korrelierter biologischer Schäden stellt die Berechnung der Anzahl von Doppelstrangbruch-Paaren (DSB-Paare) innerhalb eines vorgegebenen Abstandes dar.

Bei bisher bekannten Verfahren werden die Anzahl und die räumliche Verteilung der produzierten Schäden in der Regel nicht auf einer (Sub)-Nanometer-Skala berechnet, sondern in Mikrometer-Dimension. Die Berechnung beruht damit im Allgemeinen auf einer globalen Information der Energiedeposition über die entsprechenden Mikrometer-Bereiche; dabei wird die eigentlich auf der (Sub)-Nanometer-Skala lokalisierte physikalische Dosisdeposition im Bestrahlungsfeld zu sehr vereinfacht. Darüber hinaus basieren die bisher bekannten Verfahren in der Regel nicht auf der Übertragung der Effekte aus der Photonen-Dosiseffektkurve.

Die Kombination der präzisen Berechnung der räumlichen Schadensverteilung (rSv) im Submikrometerbereich mit der Bezugnahme auf die Photonen-Dosiseffektkurve stellt eine wesentliche Neuerung des hier vorgeschlagenen Verfahrens dar.

Der Vorteil dieses Verfahrens zur Bestrahlungsplanung liegt insbesondere darin, dass damit eine genauere Berechnung der effektiven Dosis möglich ist und damit die Bestrahlungsplanung mit einer deutlich verbesserten Genauigkeit durchgeführt werden kann. Insbesondere wird durch das Verfahren die Genauigkeit bei leichten Ionen verbessert, sodass das vorgeschlagene Verfahren mit gleicher Genauigkeit über einen großen Bereich von Ionen von Protonen bis hin zu schwereren Ionen, wie beispielsweise Neonionen, anwendbar ist. Außerdem kann damit die Wirkung von Ionenstrahlen sowohl prospektiv bei der Bestrahlungsplanung als auch retrospektiv für die Nachrechnung, Überprüfung und Validierung bereits aufgestellter und applizierter Bestrahlungspläne berücksichtigt werden.

Das Verfahren ist ebenfalls zur Bestimmung der Wirksamkeit von Neutronenstrahlen anwendbar. Bei der Bestrahlung eines Zielvolumens mit Neutronen können durch Kernreaktionen geladene Sekundärteilchen erzeugt werden. Diese verursachen wiederum Schäden im sensitiven Volumen. Die Wirkung der Neutronen beruht dabei auf der Wirkung der entstehenden so genannten "recoils" und ist damit durch ein gemischtes Teilchenfeld charakterisiert, das typischerweise geladene Teilchen von Protonen bis Sauerstoffionen enthalten kann. Zur Berechnung der Wirkung dieses gemischten Strahlenfeldes können deshalb analog zur Situation in der Ionenstrahltherapie dieselben Methoden angewendet werden.

Das vorstehend gesagte gilt analog ebenfalls für andere Teilchen.

In einer Ausgestaltung des vorgeschlagenen Verfahrens wird das Verfahren zur Erstellung eines Bestrahlungsplans für das Zielvolumen und/oder zur Validierung eines Bestrahlungsplans verwendet. Der Bestrahlungsplan wird in der Regel vor der eigentlichen Bestrahlung eines Zielvolumens ermittelt, indem ein Parameterdatensatz berechnet wird, der zur Steuerung des Bestrahlungsverfahrens in einer Bestrahlungsvorrichtung gespeichert werden kann. Der Bestrahlungsplan umfasst folglich in einer Speichereinheit in Form eines Datensatzes oder einer Wertetabelle hinterlegte Parameterdatensätze, welche direkt oder indirekt zur Steuerung einer Bestrahlungsanlage eingesetzt werden, um den Bestrahlungsplan bei der Bestrahlung umsetzen. Der Bestrahlungsplan kann gewährleisten, dass im Zielvolumen die gewünschte effektive Dosis appliziert wird. Dadurch, dass bei der ermittelten effektiven Dosisverteilung zumindest teilweise Daten verwendet werden, die von der spezifischen Strahlenreaktionen des bestrahlten oder des zu bestrahlenden Materials abhängen, kann eine Bestrahlungsplanung bzw. Bestrahlungsplan-Validierung entsprechend dem vorgeschlagenen Verfahren generell präziser durchgeführt werden. Im speziellen Fall einer Tumorbestrahlung kann damit die effektive Dosis im Tumor optimiert werden und dabei das umliegende gesunde Gewebe optimal geschont werden.

In einer Ausgestaltung des Verfahrens zur Ermittelung einer effektiven Dosisverteilung wird aus der Fluenzverteilung zumindest teilweise eine mikroskopische Dosisverteilung ermittelt. Hierbei kann die mikroskopische Dosisverteilung, insbesondere die lokale Dosisverteilung, zumindest unter teilweiser Verwendung der radialen Dosisverteilung um eine einzelne Ionenspur bestimmt werden. Die radiale Dosisverteilung beschreibt den Erwartungswert der lokalen Energiedeposition als Funktion des Abstandes von der Trajektorie der Ionenspur. Der Vorteil der Verwendung der radialen Dosisverteilung liegt darin, dass damit direkt auf die Effekte nach Photonenbestrahlung Bezug genommen werden kann. Die radiale Dosisverteilung kann beispielsweise durch Monte Carlo Simulationen erzeugt werden. Eine weitere Möglichkeit besteht durch eine analytische Dosisbeschreibung einer amorphen Bahnstruktur.

In einer weiteren Ausgestaltung des Verfahrens wird die mikroskopische, räumliche Schadensverteilung (rSv) zumindest teilweise aus der mikroskopischen Dosisverteilung (mDv) ermittelt, welche durch den Partikestrahl bzw. durch die induzierte Fluenz-/Energieverteilung hervorgerufen werden kann, wobei die dafür benötigte Wahrscheinlichkeit einer lokalen Schadensinduktion zumindest teilweise aus einer zugehörigen Photonendosiseffekt-Kurve (PEK1) abgeleitet wird. Die räumliche Schadensverteilung kann insbesondere durch die räumliche Verteilung der Schadensereignisse bestimmt sein. Die Photonendosiseffektkurven an sich sind bekannt und werden in der Regel experimentell ermittelt. Beispielsweise kann die mittlere Anzahl an Schäden häufig durch einen linear-quadratischen Zusammenhang beschrieben werden: $N_{Schaden} = \gamma D_x + \delta D_x$. $D_x$ ist hierbei die Röntgen- oder Photonendosis und $\gamma$ und $\delta$ sind materialspezifische Konstanten, wie etwa Polymerisations-Ereignisse in einem Monomer-Kristall eines GafChromic-Films oder Parameter zur Ausbeute von Doppelstrangbrüchen.

Die Wirkung von Photonenstrahlen ist dadurch charakterisiert, dass die räumliche Verteilung der entstehenden Schäden auf Grund der physikalischen Eigenschaften der Energiedeposition von Photonen gleichförmig stochastisch verteilt ist. Wenn das sensitive Volumen in Teilvolumina, die auch als Parzellen bezeichnet werden, aufgeteilt wird, kann man davon ausgehen, dass jede Parzelle die gleiche Schadensdichteverteilung aufweist. Dies bedeutet, dass eine gleichmäßige Schadensverteilung in jeder Parzelle vorliegt.

Für ein mit Ionen bestrahltes sensitives Volumen hingegen, das in Parzellen aufgeteilt ist, liegt aufgrund der extrem lokalisierten Energiedeposition von Ionenstrahlen, eine heterogene Schadensverteilung vor. Wird das sensitive Volumen in Parzellen mit einer Größe von kleiner oder kleiner/gleich 1x1x1 nm$^3$ ($\leq$1x1x1 nm$^3$) aufgeteilt und in jeder Parzelle die mikroskopische Dosis, insbesondere lokale mikroskopische Dosisverteilung bestimmt, kann somit eine mikroskopische, räumliche Schadensverteilung rSv ermittelt werden.

In einer weiteren bevorzugten Ausgestaltung des Verfahrens wird zumindest teilweise aus der räumlichen, mikroskopischen Schadensverteilung der Erwartungswert für die Anzahl korrelierter Schäden (AkS) in einem geeignet gewählten Teilvolumen eines sensitiven Volumens genauer bestimmt. Hierbei ist mit dem Begriff "korrelierter Schaden", ein Schaden gemeint, der durch das räumliche Zusammenwirken von Einzelschäden entstehen kann. Die räumliche Korrelation kann dabei durch eine Analyse der Abstände der Einzelschäden definiert werden. Im Falle von biologischem Material kann ein korrelierter Schaden zum Beispiel durch die Kombination von zwei Einzelstrangbrüchen der DNA definiert sein, die zu einem DNA-Doppelstrangbruch führen oder zwei Doppelstrangbrüchen, die zu einem Schaden führen, der für das biologische Material schlechte zu reparieren ist. Der Vorteil der Verwendung korrelierter Schäden liegt darin begründet, dass damit insbesondere die nicht-lineare Reaktion von Materialien oder Zellen auf Bestrahlung besser berücksichtigt werden kann.

In einer weiteren bevorzugten Ausgestaltung des Verfahrens zur Bestimmung der Wirkung des Partikelstrahls auf ein zumindest teilweise bestrahltes oder zu bestrahlendes Material ist das Material im Bestrahlungsvolumen zumindest teilweise ein biologisches Material. Das Material kann hierbei ein aus Zellen aufgebautes Material, Zellkulturen und/oder Gewebe, beispielsweise Tumorgewebe, umfassen. Das Volumen kann aber gleichzeitig auch anderes Material enthalten, beispielsweise ein Metall-Implantat zusammen mit biologischem Gewebe enthalten.

In einer weiteren bevorzugten Ausgestaltung des Verfahrens weist das sensitive Volumen zumindest teilweise mindestens ein Unter- und/oder Teilvolumen des biologischen Materials, insbesondere eine Zelle, auf. Das sensitive Volumen weist insbesondere ein Teilvolumen einer Zelle auf. Es kann bevorzugt ein Zellkern sein. Die Ausdehnung des sensitiven Volumens zur Bestimmung der lokalen, mikroskopischen Dosisverteilung kann demnach den typischen Dimensionen einer Zelle entsprechen, also in etwa 10 $\mu$m. Für die Unterteilung des sensitiven Volumens in die oben beschriebenen Parzellen kann die Auflösung hierbei im Bereich von Nanometern liegen, d.h. die Dimension einer der weiter oben beschriebenen Parzellen liegt in der Größenordnung von typischerweise $\leq$1 nm. Der Vorteil der Betrachtung auf der Nanometer-Skala liegt darin begründet, dass bei einer hinreichend feinen Auflösung die mikroskopische Dosis innerhalb einer Parzelle in erster Näherung als konstant angenommen werden kann. Diese Annahme erlaubt es, die Wirkung der Energiedeposition von Ionenstrahlen innerhalb einer Parzelle aus der DosisEffektkurve für Photonenstrahlung abzuleiten.

In einer weiteren Ausgestaltung des Verfahrens wird eine Photonendosis (PD1) ermittelt, die zum Erreichen derselben Ausbeute korrelierter Schäden, d.h. derselben Anzahl korrelierter Schäden (AkS) bezogen auf die Gesamtzahl der Einzelschäden oder der isolierten und korrelierten Schäden, wie nach Ionenbestrahlung erforderlich gewesen wäre. Das bedeutet, dass zu einer makroskopischen Photonendosis innerhalb des Bestrahlungsfeldes eine räumliche Verteilung der Schäden, deren Art und deren Anzahl gemäß der Photonen-Dosiseffektkurve PEK1, ermittelt wird, aus der der Erwartungswert der Anzahl der korrelierten Schäden bestimmt werden kann. Insbesondere ergibt sich somit einer Photonendosis PD1, die zur gleichen Ausbeute korrelierter Schäden wie nach Ionenstrahlung führt. Beispielsweise kann man für den Erwartungswert der Anzahl von Doppelstrangbruchpaaren innerhalb eines vorgegebenen Abstands annehmen, dass dieser quadratisch von der makroskopischen Photonendosis abhängt. Insbesondere ermöglicht dies eine schnelle Bestimmung von PD1 basierend auf der Simulation der Ausbeute von korrelierten Schäden bei einer bestimmten Photonendosis. Dazu wird angenommen, dass bei genügend klein gewählten Parzellen und bei gleicher lokaler Dosis mit Photonen- und mit Ionenbestrahlung ein gleicher Schaden induziert wird. Dies kann auch damit begründet werden, dass bei einem genügend kleinem Parzellen-Volumen auch im Falle der Ionenstrahlen der Erwartungswert der Energiedeposition in diesem Volumen als homogen verteilt angenommen werden kann und damit direkt mit der Wirkung der Bestrahlung von Photonenstrahlen verglichen werden kann. Hierbei ist vorteilhaft, dass die Anzahl der Schäden, die aus Photonenbestrahlung bekannt sind, auf ein mit Ionen bestrahltes Material in dieser Dimension übertragen werden können.

In einer weiteren Ausgestaltung des Verfahrens wird aus einer zweiten Photonendosiseffektkurve für den beobachtbaren Effekt (PEK2) zumindest teilweise ein zu der Photonendosis (PD1) gehöriger Effekt (E1) bestimmt. Im Gegensatz zur Photonen-Dosiseffektkurve (PEK1), die den lokalen, mikroskopischen bzw. molekularen Schaden beschreibt, repräsentiert die Photonen-Dosiseffektkurve (PEK2) den makroskopisch beobachtbaren Effekt. Im Falle von Zellen kann PEK2 z.B. die Inaktivierung von Zellen beschreiben, beispielsweise charakterisiert durch die linear-quadratischen Parameter $\alpha_x$ und $\beta_x$, die für viele Zell- und Gewebearten bekannt sind. Im Falle von Geweben bzw. Organen kann PEK2 die Wahrscheinlichkeit von Gewebeschädigungen bzw. Organversagen beschreiben. Ein Vorteil liegt ähnlich wie bei PEK1 darin, dass durch Bezugnahme auf die experimentellen Photonen-Daten, PEK2 eine hohe Genauigkeit der ermittelten biologischen Effekte für Ionenbestrahlung erreicht werden kann. Ferner können mit diesem Verfahren im Vergleich zu anderen Verfahren die Rechenzeiten drastisch reduziert werden.

In einer weiteren bevorzugten Ausgestaltung des Verfahren wird der beobachtbare Effekt (E2) für die gegebene Fluenzverteilung zumindest teilweise aus der Skalierung des biologisch relevanten Effekts (E1) entsprechend dem Verhältnis aus der Photonendosis (PD1) und der im sensitiven Volumen entsprechend der Fluenzverteilung deponierten Dosis (ID) bestimmt. Insbesondere kann der beobachtbare Effekt (E2) wie folgt berechnet werden:

$$E2 = E1 \cdot \frac{ID}{PD1} \text{ oder } E2 = E1 \cdot \frac{AkS_2}{AkS_1} \quad (6)$$

wobei beispielsweise angenommen werden kann, dass E2 durch ein einzelnes Ion entstanden ist mit zugehöriger Dosisdeposition ID und zugehöriger PD1 entstanden ist. Alternativ kann bevorzugt angenommen werden, dass E2 durch ein einzelnes Ion entstanden ist und AkS1 bzw. AkS2 die Anzahl korrelierter Schäden durch die Photonendosis PD1 bzw. die Ionendosis ID beschreiben.

Alternativ kann die Wirkung des Partikelstrahls auch direkt aus der Anzahl der isolierten und korrelierten Schäden bestimmt werden, sofern der Effekt dieser Schadenstypen aus der Photonendosiseffektkurve abgeleitet werden kann:

$$E2 = AiS2 \cdot \varepsilon_i + AkS2 \cdot \varepsilon_k \quad (7)$$

Dabei bezeichnet AiS2 die Anzahl isolierter Schäden und AKS2 die Anzahl korrelierter Schäden, die durch ein Ion induziert werden können; $\varepsilon_i$ sowie $\varepsilon_k$ beschreiben die Wirkung einzelner isolierter bzw. korrelierter Schäden.

In einer weiteren Ausgestaltung des Verfahrens wird die effektive Dosis zumindest teilweise aus der dem beobachtbaren Effekt (E2) zugehörigen Photonendosis (PD2) bestimmt. Durch geeignete Optimierungsverfahren kann damit die Ionendosis für jeden Punkt des Bestrahlungsfeldes so gewählt werden, dass damit ein vorgegebener Effekt in jedem Punkt des Bestrahlungsvolumens erzielt werden kann. Im Falle eines gemischten Strahlenfeldes kann beispielsweise PD2 auch aus der Betrachtung des Strahlenfeldes an jedem Punkt des Bestrahlungsfeldes gemäß der im Stand der Technik bekannten Methoden bestimmt werden. Dabei kann insbesondere die so genannte intrinsische RBW von einzelnen Ionen benutzt werden, wobei sich die intrinsische RBW wie folgt ermitteln lässt:

$$RBW_{int} = \frac{\alpha_{int}}{\alpha_X} = \frac{E2_{Single}}{ID_{single} \cdot \alpha_X}, \quad (8)$$

wobei $\alpha_{int}*ID_{single}$ dem biologischen Effekt $E2_{single}$ eines einzelnen Ions mit Dosisdeposition $ID_{single}$ entspricht

Gemäß der Veröffentlichung (Krämer und Scholz, Physics in Medicine and Biology, Vol. 51, S 1959- 1970, 2006.) lässt sich $\beta_{int}$ aus $\alpha_{int}$ gewinnen und aus $\alpha_{int}$ und $\beta_{int}$ für das gesamte gemäß der Fluenzverteilung erzeugte gemischte Strahlenfeld letztlich der Effekt E2. Dabei kann der Effekt nach Ionenbestrahlung beispielsweise mittels der linear-quadratischen Parameter $\alpha_{ion}$ und $\beta_{ion}$ beschrieben werden.

Der Vorteil des Verfahrens ist darin zu sehen, dass es eine direkte Übertragung der Erfahrungen mit konventioneller Bestrahlung auf die Bestrahlung mit Teilchenstrahlen erlaubt. Ein weiterer Vorteil des vorgeschlagenen Verfahrens besteht darin, dass die RBW_{int}-Werte im Voraus unabhängig vom aktuellen Strahlenfeld für einzelne Ionen von Protonen bis Neon für Energien von 0.1 MeV/u bis 1 GeV/u vorherberechnet und in Form von Tabellen gespeichert werden können, bevor E2 für das gemischte Strahlenfeld berechnet wird. Dies kann zu einer weiteren erheblichen Ersparnis an Rechenzeit führen.

Die Aufgabe kann ferner durch ein Verfahren zur Bestrahlung eines Zielvolumens mit einem Partikelstrahl gelöst werden, welches jedoch nicht Teil der Erfindung ist, aufweisend die Schritte: - Ermitteln einer Fluenz- und/oder Energieverteilung innerhalb des, das Zielvolumen (44) aufweisende Bestrahlungsvolumens (46); - Ermitteln einer aus der Fluenz- und/oder Energieverteilung resultierenden effektiven Dosisverteilung, wobei die Wirkung des Partikelstrahls im Material des Bestrahlungsvolumens (46) verwendet wird, welche zumindest teilweise auf der Basis der mikroskopischen Schadenskorrelation nach einem Verfahren zur Bestimmung der Wirkung eines Partikelstrahls in einem Material.

Die Eigenschaften und Merkmale sowie Vorteile für das Verfahren zur Bestrahlungsplanung gelten in analoger Art und Weise für das Verfahren zur Bestrahlung eines Zielvolumens.

Die Aufgabe wird ferner durch einen Bestrahlungsplan zur Bestrahlung eines ein Zielvolumen aufweisenden Bestrahlungsvolumens gelöst, wobei der Bestrahlungsplan zumindest teilweise unter Verwendung des vorstehend beschriebenen Verfahrens aufstellbar ist und/oder aufgestellt wurde. Bei der Erstellung des Bestrahlungsplans werden demzufolge Daten verwendet, die die aus einer Fluenzverteilung resultierende effektive Dosisverteilung bestimmt, wobei der relevante, beobachtbare Effekt im Material des Bestrahlungsvolumens zumindest teilweise auf der Basis der makroskopischen Schadenskorrelation, als auch insbesondere auf Basis der Anzahl korrelierter Schäden (AkS), ermittelt wird.

Die Aufgabe wird ferner durch eine Strahlmodifikationseinrichtung gelöst, die unter Verwendung des vorstehend beschriebenen Verfahrens zur Bestrahlung eines Zielvolumens herstellbar und/oder betreibbar ist. Die Strahlmodifikationseinrichtung kann hierbei eine aktive und/oder passive Vorrichtung aufweisen, die zum Durchführen des Verfahrens eingerichtet ist. Eine aktive Strahlmodifikationseinrichtung kann hierbei beispielsweise eine Rasterscan-Einrichtung sein, die den Teilchenstrahl gemäß eines Orts- und Energieparameterfeldes über das Zielvolumen führt. Eine passive Strahlmodifikationseinrichtung kann hierbei beispielsweise ein Filterelement sein, das mittels des vorgeschlagenen Verfahrens entworfen oder hergestellt wurde. Das Filterelement, das auf diese Art und Weise entworfen wurde, kann dann in eine Bestrahlungseinrichtung eingebaut werden. Hierbei kann mittels des Filterelementes eine Energie und damit die Eindringtiefe (z-Richtung) des Ionen- oder Partikelstrahls modifiziert werden, während jeweils die x- und die y-Richtung durch Ablenkung des Strahls mittels eines Scanners abgefahren wird. Somit kann durch Zusammenwirken des passiven Filterelementes und des aktiven Scanners das zu bestrahlende Zielvolumen abgescannt werden.

Die Aufgabe wird ferner durch eine Bestrahlungsvorrichtung gelöst, die mindestens eine Strahlmodifikationseinrichtung aufweist, die die vorstehend beschriebenen Merkmale umfasst. Hierbei kann die Bestrahlungsvorrichtung insbesondere eine Beschleunigervorrichtung zum Erzeugen und Beschleunigen eines Partikelstrahls umfassen sowie eine Strahlmodifikationseinrichtung aufweisen, mittels der ein zu bestrahlendes Zielvolumen in allen drei Raumrichtungen abgetastet werden kann. Hierbei kann die Strahlmodifikationsvorrichtung sowohl passive als auch aktive als auch eine Kombination von passiven und aktiven Einrichtungen zur Änderung der Strahlenergie und Strahllage und/oder Strahlachse am Ort des Zielvolumens aufweisen. Die Bestrahlungsvorrichtung weist bevorzugt ein Kontrollsystem zur Steuerung der Beschleunigervorrichtung und mindestens eine Strahlmodifikationseinrichtung zum Bestrahlen von Gegenständen, insbesondere Patienten, auf. Hierdurch kann die Bestrahlungsvorrichtung mittels des Kontrollsystems gesteuert werden, wobei das Kontrollsystem eine Einrichtung aufweist, in der ein Bestrahlungsplan gespeichert ist oder mit der ein entsprechender Bestrahlungsplan erzeugt werden kann.

Die Erfindung wurde in Bezug auf bevorzugte Ausführungsformen beschrieben und es wird betont, dass Äquivalente, Alternativen und Modifikationen der Gegenstände und/oder Merkmale im Umfang des Schutzbereiches enthalten sind, solange diese nicht außerhalb des Schutzumfangs der Ansprüche liegen.

Nachfolgend wird die Erfindung anhand der Figuren der Zeichnung näher beschrieben. Hierbei werden für ähnliche Gegenstände in den Figuren gleiche Bezugszeichen verwendet. Es zeigen:

Fig. 1 eine schematische Darstellung eines Funktionsprinzips einer Strahlmodifikationseinrichtung und deren Wirkung auf eine Tiefendosisverteilung;

Fig. 2 eine schematische Darstellung einer Bestrahlungsvorrichtung mit einer aktiven Strahlmodifikationseinrichtung (Fig. 2a) und mit einer passiven Strahlmodifikationseinrichtung;(Fig. 2b)

Fig. 3 eine schematische Darstellung einer mikroskopischen Dosisverteilung in einem sensitiven Volumen für Bestrahlung mit Ionen (3a, 3b, 3c) und mit Photonen (3d);

Fig. 4 ein Ablaufdiagramm eines Verfahrens zur Bestrahlung eines Zielvolumens;

Fig. 5 ein Ablaufdiagramm von Verfahrensschritten, die in dem in Fig. 4 gezeigten Verfahrens näher ausgeführt werden können;

Fig. 6 eine Verteilung von DNA-Schäden in einem sensitiven Volumen nach einer Bestrahlung mit Ionen und Photonen;

Fig. 7        eine Verteilung von DNA-Schäden in einem sensitiven Volumen nach Photonenbestrahlung, die zur gleichen Ausbeute korrelierter Schäden wie die in der Fig. 6 gezeigte Bestrahlung mit Ionen führt;

Fig. 8        eine Funktion eines RBW-Faktors in Abhängigkeit vom linearen Energietransfer (LET);

Fig. 9a       eine schematische Darstellung einer mit Heliumionen applizierten Bestrahlungsdosis in einem Zielvolumen als Funktion der Eindringtiefe;

Fig. 9b       eine Darstellung eines Zellüberlebens als Funktion der Eindringtiefe in einem Zielvolumen nach Bestrahlung mit dem Bestrahlungsfeld aus Fig. 9a;

Fig. 10a      eine schematische Darstellung einer mit Protonen und Kohlenstoffionen applizierten Bestrahlungsdosis in einem Zielvolumen für den Fall einer typischen Zwei-Feld Bestrahlung

Fig. 10b      eine schematische Darstellung des berechneten Zellüberlebens und der zugehörigen experimentell ermittelten Überlebens-Wahrscheinlichkeiten für die Bestrahlung eines Zielvolumens mit den Bestrahlungsfeldern aus 10a.

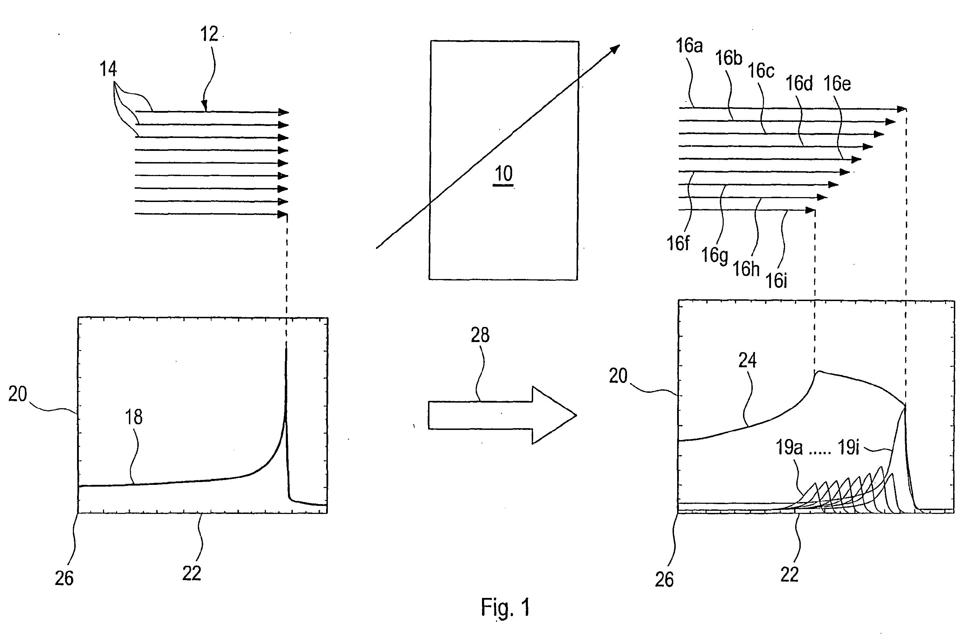

Fig. 1 stellt in schematischer Darstellung eine Strahlmodifikationseinrichtung 10 und deren Wirkung auf einen monoenergetischen Ionen- oder Partikelstrahl 12 dar. Der monoenergetische Ionenstrahl 12 ist mittels Pfeilen 14 mit gleicher Länge schematisch gezeigt. Die Pfeile 16a bis 16i stellen Ionenstrahlen unterschiedlicher Energie dar und somit einen Ionenstrahl 16, der nicht monoenergetisch ist, also Ionen unterschiedlicher Energie aufweist. Im unteren Teil der Fig. 1 ist jeweils in schematischer Darstellung ein Diagramm gezeigt, welches einen Bragg-Peak 18' (linke Darstellung), 24 (rechte Darstellung) in einem zu bestrahlenden Material (nicht dargestellt) zeigt, der die relative Dosis als Funktion der Eindringtiefe im Material zeigt. Die relative Dosis ist auf einer als y-Achse bezeichneten Achse 20 aufgetragen und die Eindringtiefe auf einer als x-Achse bezeichneten Achse 22. Der Kurvenverlauf des Bragg-Peaks 18 beschreibt den Verlauf der Dosis als Funktion der Tiefe für eine Bestrahlung mit monoenergetischen Ionen. Der Bragg-Peak 24 beschreibt die relative Dosis im Material als Funktion der Eindringtiefe für einen Ionenstrahl 16 mit den durch die Pfeile 16a bis 16i dargestellten unterschiedlichen Energien. Der Bragg-Peak 24 wird als extended Bragg-Peak 24 bezeichnet und setzt sich aus einer Überlagerung von einzelnen Bragg-Peaks 19a bis 19i für jeweils eine durch die Pfeile 16a bis 16i symbolisch dargestellte Energie des Ionenstrahls 16 zusammen. Der Ionenstrahl 16 kann hierbei mittels der Strahlmodifikationseinrichtung 10 aus dem Ionenstrahl 12 erzeugt werden. Die relative Dosis entspricht hierbei einer deponierten Dosis in einem Bestrahlungsvolumen (nicht dargestellt). Der Nullpunkt 26 der x-Achse 22 entspricht der Oberfläche des Bestrahlungsvolumens.

Hierbei ist schematisch die Wirkung der Strahlmodifikationseinrichtung 10 gezeigt, mit deren Hilfe ein Zielvolumen (nicht dargestellt) mit einer vorgegebenen Dosisverteilung bestrahlt werden kann. Dazu wird ein monoenergetischer Strahl durch aktive oder passive Strahlführungselemente der Strahlmodifikationseinrichtung 10 bezüglich der Strahlenergie und Eindringtiefe so moduliert, dass damit ein vorgegebener Tiefenbereich mit der gewünschten Dosis belegt werden kann. Der Pfeil 28 symbolisiert die Wirkung der Strahlmodifikationseinrichtung 10 ausgehend von dem Bragg-Peak 18 für monoenergetische Ionenbestrahlung hin zu dem Bragg-Peak 24 für den Ionenstrahl 16.

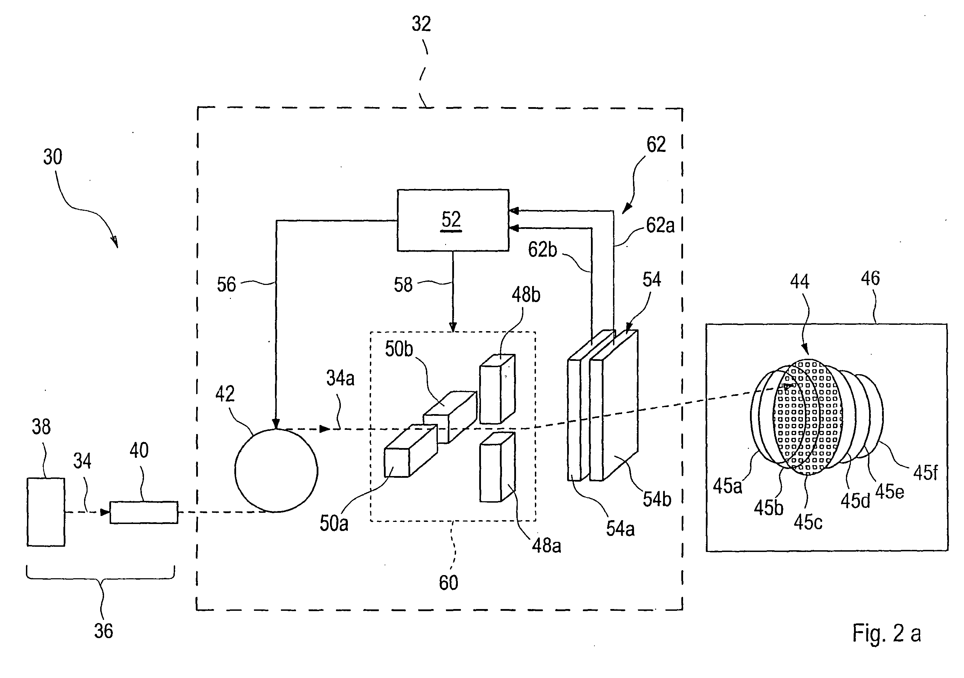

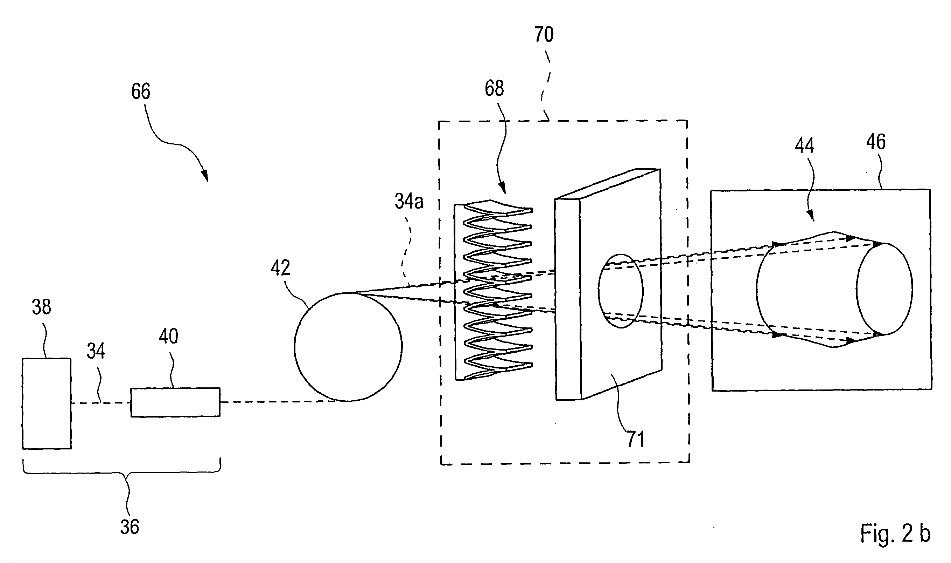

Fig. 2 stellt verschiedene Realisierungsmöglichkeiten der Strahlmodifikationseinrichtung 10 dar. Fig. 2a zeigt in schematischer Darstellung einen Aufbau einer als Bestrahlungsvorrichtung oder Partikeltherapieanlage 30 aufgebauten Beschleunigereinrichtung 36 mit einer aktiven Strahlmodifikationseinrichtung 32, die eine aktive Strahlführung und Strahlapplikation umfasst. Die Partikeltherapieanlage 30 weist typischerweise eine einen Partikelstrahl 34 erzeugende Strahlerzeugungseinrichtung 38 und eine Strahlbeschleunigungseinrichtung 40 auf. Die Strahlerzeugungseinrichtung 38 weist typischerweise eine Partikelquelle, beispielsweise eine Ionenquelle, auf. Die Strahlbeschleunigungseinrichtung weist typischerweise eine Niederenergiebeschleunigereinheit und eine zugehörige Strahlführung auf. Der Beschleunigereinrichtung 36 nachgeschaltet ist ein Synchrotron 42, ein Zyklotron oder einen sonstigen Beschleuniger sowie eine Hochenergiestrahltransporteinrichtung auf. Nach dem Synchrotron 42 wird ein Partikelstrahl 34a mit der zur Bestrahlung notwendigen Energie bereitgestellt. Als Partikel werden Typischerweise Partikel oder Teilchen wie Protonen, Pionen, Heliumionen, Kohlenstoffionen, Sauerstoffionen oder andere als Ionen bezeichnete geladene Teilchen oder Partikel chemischer Elemente oder Verbindungen verwendet. Somit werden die Begriffe Ionen, Teilchen und Partikel synonym in der Erfindung verwendet.

In Fig. 2 ist beispielhaft die Ionenquelle 38, ein Linearbeschleuniger 40 und ein Synchrotron 42 gezeigt. Die Beschleunigereinrichtung 36 und die nachgeschaltete Hochenergiebeschleunigungseinrichtung kann auch jeden anderen Beschleuniger aufweisen, der in der Lage ist, einen Partikelstrahl 34a, insbesondere aus geladenen Ionen, mit der für die Bestrahlung eines Zielvolumens 44 notwendigen Energie bereitzustellen. Für die Anwendung in der Tumortherapie werden typischerweise Beschleunigereinrichtungen 36 eingesetzt, die einen Ionenstrahl 34a mit einer Maximalenergie der Partikel in der Größenordnung von typischerweise 1 GeV /amu bereitstellen.

Das Zielvolumen 44 ist in einem Objekt oder Bestrahlungsvolumen 46 angeordnet, wobei das Objekt 46 das das Zielvolumen 44 umgebende Volumen umfassen kann und/oder den gesamten zu bestrahlenden Gegenstand. Das zu bestrahlende Zielvolumen 44 kann ein beliebig geformtes Volumen sein, das der Partikelstrahlung ausgesetzt werden soll, zum Beispiel ein Filmdetektorsystem, ein mit Zellkulturproben gefülltes Volumen oder ein Tumorvolumen in einem Patienten (nicht dargestellt). Das Zielvolumen 44 kann sowohl ein ruhendes Zielvolumen 44 als auch ein sich bewegendes Zielvolumen 44 sein. Das Zielvolumen 44 wird typischerweise in Scheiben 45a, 45b, 45c, 45d, 45e, 45f aufgeteilt, die jeweils mit einem Ionenstrahl 34a mit einer für die jeweilige Scheibe 45a, 45b, 45c, 45d, 45e und 45f notwendigen spezifischen Energie bestrahlt werden. Die jeweilige Energie wird üblicherweise mit dem Synchrotron 42 eingestellt und ist variierbar.

Zum Applizieren der Gesamtdosis in dem zu bestrahlenden Zielvolumen 44, wird das Zielvolumen 44 beispielsweise mittels eines Rasterscan-Verfahrens bestrahlt, Dabei wird ein dünner Bleistiftstrahl mithilfe von Ablenkmagneten 48a und 48b sowie 50a und 50b lateral über das zu bestrahlende Volumen 46 gelenkt, durch Variation der Hochenergie-Beschleunigereinstellung oder der Absorberdicke wird die Strahlenergie und somit die Eindringtiefe des Strahls 34a variiert und der Strahl damit in longitudinaler Richtung über das zu bestrahlende Volumen, insbesondere über die Scheiben 45a, 45b, 45c, 45d, 45e und 45f geführt.

Die Bestrahlungsvorrichtung 30 weist dazu eine Ablaufsteuereinrichtung 52 sowie mindestens einen Detektor 54 zur Überwachung der als Ionenstrahlparameter bezeichneten Parameter des Ionenstrahls 34a auf. Die Ablaufsteuerungseinrichtung 52 ist mittels einer typischerweise elektrischen Verbindung 56 mit der Beschleunigereinrichtung 36, insbesondere dem Synchrotron 42, und mittels einer Verbindung 58 mit einer Rasterscaneinrichtung 60 gekoppelt. Mittels einer Verbindung 62 ist die Ablaufsteuerung 52 mit dem Detektor 54 verbunden. Somit können über den Detektor 54 ermittelten Werte (Parameter des Partikelstrahls 34a), wie beispielsweise Energie und Position des Partikelstrahls 34a, zur Steuerung und Regelung der Bestrahlungsvorrichtung 36, insbesondere des Synchrotrons 42, verwendet werden. Der Detektor 54 kann aus mehreren Detektoreinheiten aufgebaut sein. In der gezeigten Ausführungsform ist der Detektor 54 aus zwei Detektoreinheiten 54a und 54b zusammengesetzt und folglich weist die Verbindung 62 zwei Verbindungen 62a und 62b auf. Ein Beispiel für einen Detektor 54 zur Bestimmung der Ionenstrahlparameter ist zum Beispiel eine Ionisationskammer 54a und eine Vieldraht-Proportionalkammer 54b, die als Strahlintensitäts- oder Strahlpositions-Monitore einsetzbar oder geeignet sind.

Die Ablaufsteuereinrichtung 52 stellt das Kontrollsystem der Partikelstrahlanlage 30 dar und steuert somit die einzelnen Komponenten der Anlage 30, wie beispielsweise die Beschleunigereinrichtung 36 , das Synchrotron 42 und die Magnete 48a, 48b sowie 50a und 50b der Rasterscan-Einrichtung 60. Ferner können in der Ablaufsteuereinrichtung 52 Messdaten, wie beispielsweise Daten des Detektors 54, zur Überwachung der Strahlparameter eingelesen und/oder gespeichert werden.

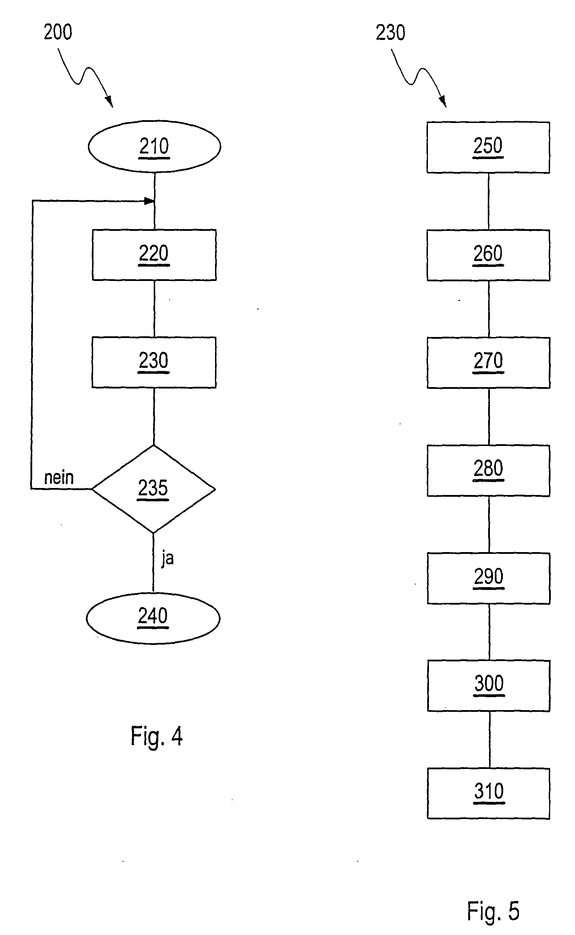

Üblicherweise erfolgt die Steuerung der Strahlparameter der Partikelstrahlanlage 30 mit Hilfe eines Bestrahlungsplans, der vor einer Bestrahlung erzeugt wird. Dieser Bestrahlungsplan wird typischerweise vor dem Beginn der Bestrahlung des Zielvolumens 44 in einer Planungseinrichtung erstellt. Es kann aber auch vorgesehen sein, den Bestrahlungsplan zu erstellen und/oder zu modifizieren, wenn die Bestrahlung bereits begonnen hat. Mittels eines Verfahrens 200 (in Fig. 4 dargestellt) können derartige Bestrahlungspläne erstellt werden.

Die in Fig. 2a gezeigte Strahlmodifikationseinrichtung ist eine aktive Strahlmodifikationseinrichtung 32. Die Anordnungen der in Fig. 2a gezeigten Komponenten der Partikelstrahlanlage 30 sind lediglich beispielhaft. Auch andere Anordnungen, insbesondere andere Komponenten zur Strahlerzeugung und zur Strahlmodifikationen sind einsetzbar.

Fig. 2b zeigt in schematischer Darstellung ein Beispiel einer anderen Ausführungsform einer Bestrahlungseinrichtung 66. Die Modulation der Strahlenergie (Energie des Ionenstrahls) erfolgt in diesem Falle über ein sog. Ridge-Filter-System 68. Die Form dieses Filters 68 ist so ausgelegt, dass durch variable Dicke an verschiedenen Stellen des Filters ein Ionenstrahl 34a unterschiedlich stark abgebremst wird. Durch die Konstruktion des Filters 68 wird damit die genaue Form der Tiefendosisverteilung des Ionenstrahls im Zielvolumen 44 eindeutig festgelegt. Umgekehrt bestimmt eine vorgegebene Tiefendosisverteilung die Auslegung des zugehörigen Filters 68. Die technische Auslegung des Filters 68 ist somit typischerweise vom jeweiligen ermittelten Bestrahlungsplan abhängig. Das Verfahren 200 zur Erstellung eines Bestrahlungsplanes kann bei der Fertigung des Filtersystems 68 und/oder einzelner Komponenten eingesetzt werden und kann damit z.B. zur Erzeugung von Steuerdaten für CNC-Fertigungsmaschinen dienen. Die durch das vorgeschlagene Verfahren erzeugten Daten der zu erzielenden effektiven Dosisverteilung können somit bei der Herstellung des Filters 68 eingesetzt werden. Die in Fig. 2b gezeigte Strahlmodifikationseinrichtung 10 wird als eine passive Strahlmodifikationseinrichtung 70 bezeichnet. Die passive Strahlmodifikationseinrichtung 70 umfasst typischerweise neben dem Filter 68 eine Kollimatoreinrichtung 71, von der schematisch lediglich ein Kollimator dargestellt ist.

Neben den oben beschrieben aktiven und passiven Verfahren zur Strahlmodifikation sind auch gemischte Verfahren zur Implementierung einer Strahlmodifikationseinrichtung denkbar.

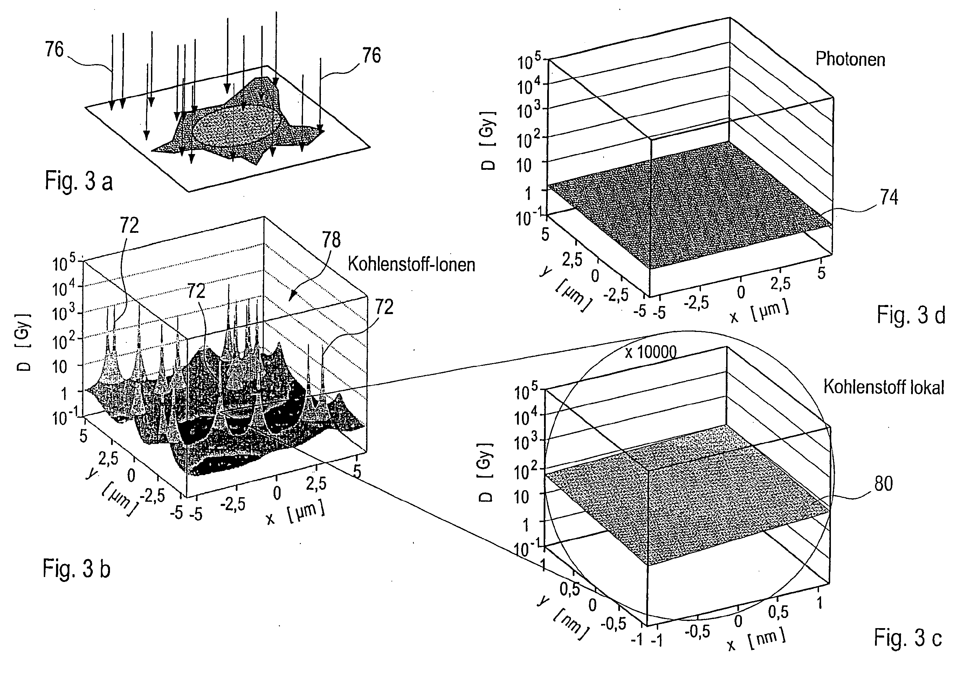

Fig. 3 zeigt schematisch den Erwartungswert der Energiedeposition auf einer Mikrometerskala für Photonenstrahlen (3d) und Ionenstrahlen (3b). Die im Vergleich zu Photonenstrahlung veränderte Wirkung von Ionenstrahlen lässt sich auf die unterschiedliche mikroskopische Verteilung der Energiedeposition 72 der verschiedenen Strahlenarten, also Ionenstrahlung und Photonenstrahlung, zurückführen. Bei Photonenstrahlung erfolgt diese Verteilung im Mittel gleichmäßig über eine betrachtete Fläche 74. Im Gegensatz dazu deponieren Ionen ihre Energie sehr heterogen verteilt. In der Nähe einer Trajektorie oder Spur 76 eines Ions (in einigen nm Abstand) können extrem hohe Dosen D (bis zu ca. $10^6$ Gy) deponiert werden, wohingegen in größeren Abständen (einige $\mu$m) von der Trajektorie 76 die Dosiswerte sehr schnell auf sehr geringe Werte (<< 1 Gy) abfallen können. Damit ergibt sich insgesamt eine sehr heterogene Verteilung 78 (3b) der deponierten Energie. Betrachtet man jedoch nur einen kleinen Teilbereich (nm$^3$) 80 (3c) dieser heterogenen Verteilung 78 so kann die in diesem Teilbereich deponierte Dosis wieder als näherungsweise konstant angenommen werden. Somit ähnelt diese Verteilung, der Verteilung, die für die Bestrahlung mit Photonen bei gleicher lokaler Dosis erwartet würde.

Somit kann durch die Auswahl eines geeigneten Teilbereichs 80 aufgrund der vorstehend beschriebenen Ähnlichkeit die Wirkung von Ionenstrahlen in einem kleinen Teilbereich aus der Wirkung von Photonenstrahlung abgeleitet werden. Insbesondere wird als Teilbereich, insbesondere als Teilvolumen, ein sensitives Volumen gewählt.

Die Ausnutzung dieses Prinzips zur Berechnung der Wirkung von Ionenstrahlen zur Ermittlung und Optimierung eines Bestrahlungsplanes wird im Folgenden anhand des in Fig. 4 dargestellten Verfahrens genauer erläutert.

Fig. 4 zeigt ein Ablaufdiagramm eines Verfahrens 200 zur Erstellung eines Bestrahlungsplanes, der zur Bestrahlung des Zielvolumens 44 mit dem Partikelstrahl 34a (vergleiche Fig. 2) verwendet wird. Bei der Bestimmung des Bestrahlungsplanes wird in Verfahrensschritt 210 mit der Berechnung begonnen. Im Verfahrensschritt 220 wird eine Fluenzverteilung in einem Zielvolumen 44 des Bestrahlungsvolumens 46 ermittelt. Das Zielvolumen 44 kann ein zu bestrahlendes anorganisches, organisches oder biologisches Material aufweisen. Biologisches Material setzt sich typischerweise aus Zellen zusammen. Das Zielvolumen 44 kann beispielsweise mindestens eine Zellkultur und/oder mindestens ein Gewebe, beispielsweise ein Tumorgewebe, aufweisen.

In Verfahrensschritt 230 wird eine aus der Fluenzverteilung resultierende effektive Dosisverteilung ermittelt, wobei Daten verwendet werden, die den im jeweiligen Material beobachtbaren Effekt auf der Basis einer mikroskopischen, insbesondere lokalen, Schadenskorrelation bestimmt. In Schritt 235 wird abgefragt, ob für die gegebene Fluenzverteilung die gewünschte Zieldosis erreicht wird. Wenn dies der Fall ist, wird das Verfahren im Schritt 240 beendet. Wenn dies nicht der Fall ist, wird die Fluenzverteilung entsprechend der Differenz zwischen Soll- und Istwert der effektiven Dosisverteilung modifiziert und dann mit Schritt 220 fortgefahren.

Das Verfahren 200 kann zur Bestrahlung des Zielvolumens 44, zur Aufstellung eines Bestrahlungsplanes, der bei der Bestrahlung des Zielvolumens 44 verwendet wird, und/oder zur Validierung des Bestrahlungsplanes vor oder nach erfolgter Bestrahlung des Zielvolumens 44 verwendet werden.

Das Verfahren 200 beinhaltet im Verfahrensschritt 230 die Verwendung eines biophysikalischen Modells, da die Anwendung der Partikelstrahlen bei der Bestrahlung von Materie, insbesondere von biologischem Gewebe in der Tumortherapie, die genaue Kenntnis der biologischen Wirkungen der Partikelstrahlen erfordern. Das verwendete biophysikalische Modell wird als Local-Effect-Model (LEM) bezeichnet und berücksichtigt die komplexe Abhängigkeit der Wirksamkeit von Parametern des Partikelstrahls 34a, wie Art der Partikel (Ionensorte), Ionenstrahlenergie, Ionendosis und Material, insbesondere Zell- beziehungsweise Gewebetyp. Hierbei wird bei der Berechnung der effektiven Dosisverteilung die biologische Wirkung der lokalen Energiedeposition innerhalb einer Zelle beziehungsweise eines Zellkerns berücksichtigt.

Fig. 5 zeigt ein Ablaufdiagramm der Verfahrensschritte, die im Verfahrensschritt 230 des Ablaufdiagramms von Fig. 4 enthalten sein können.

Im Verfahrensschritt 250 wird aus der Fluenzverteilung eine mikroskopische Dosisverteilung mDv ermittelt. Hierbei kann die mikroskopische Dosisverteilung, insbesondere die lokale Dosisverteilung, zumindest unter teilweiser Verwendung der radialen Dosisverteilung um eine einzelne Ionenspur bestimmt werden. Die radiale Dosisverteilung beschreibt den Erwartungswert der lokalen Energiedeposition als Funktion des Abstandes von der Trajektorie der Ionenspur. Der Vorteil der Verwendung der radialen Dosisverteilung liegt darin, dass damit direkt auf die Effekte nach Photonenbestrahlung Bezug genommen werden kann. Die radiale Dosisverteilung kann beispielsweise durch Monte Carlo Simulationen erzeugt werden. Eine weitere Möglichkeit besteht durch eine analytische Dosisbeschreibung einer amorphen Bahnstruktur. Die radiale Dosisverteilung beschreibt den Erwartungswert der lokalen Energiedeposition als Funktion des Abstandes von der Trajektorie der Ionenspur. Der Vorteil der Verwendung der radialen Dosisverteilung liegt darin, dass damit direkt auf die Effekte nach Photonenbestrahlung Bezug genommen werden kann. Die radiale Dosisverteilung kann beispielsweise durch Monte Carlo Simulationen erzeugt werden. Eine weitere Möglichkeit besteht durch eine analytische Dosisbeschreibung einer amorphen Bahnstruktur. in der Form, wie sie in den nach dem Stand der Technik bekannten Modellen LEM angewendet werden:

$$D_{track}(r) = \begin{cases} \lambda\, LET\,/\,r_{min}^2 & : & r < r_{min} \\ \lambda\, LET\,/\,r^2 & : & r_{min} \le r \le r_{max} \\ 0 & : & r > r_{max} \end{cases} , \quad (9)$$

wobei A eine Normierungskonstante ist, LET den linearen Energietransfer beschreibt, $r_{min}$ den inneren Bereich mit konstanter Dosis charakterisiert und $r_{max}$ der maximale Spurradius ist, beispielsweise bestimmt durch:

$$r_{max} = \gamma\, E^{\delta} \qquad (10)$$

mit $\gamma=0.062$, $\delta=1.7$. $r_{max}$ ist hierbei gegeben in $\mu$m und E ist die spezifische Energie des Ions in MeV/u. Der LET bezeichnet hierbei die von einem Ion pro Weglängeneinheit bei Durchgang durch wasseräquivalentes Material deponierte Energie und wird in keV/$\mu$m angegeben.

Im Verfahrenschritt 260 wird eine räumliche Schadensverteilung rSv zumindest teilweise aus der mikroskopischen Dosisverteilung mDv ermittelt, wobei die Wahrscheinlichkeit einer lokalen Schadensinduktion zumindest teilweise aus einer zugehörigen Photonendosiseffektkurve PEK1 abgeleitet wird. Im Falle biologischer Zielvolumens, z.B. Zellen oder Gewebe, kann die räumliche Schadenverteilung rSv zum Beispiel durch die Verteilung der Doppelstrangbrüche (DSB) im Zellkern gegeben sein. In diesem Fall ist die zugehörige Photonendosiseffektkurve PEK1 diejenige Dosiseffektkurve, die die Induktion von Doppelstrangbrüchen als Funktion der Dosis beschreibt.

Im Verfahrenschritt 270 wird der Erwartungswert für die Anzahl korrelierter Schäden in einem geeignet gewählten Volumen zumindest teilweise aus der räumlichen Schadensverteilung rSv innerhalb eines sensitiven Volumens bestimmt. Im Falle von biologischem Material weist das sensitive Volumen mindestens ein Unter- und/oder Teilvolumen einer Zelle des biologischen Materials auf. Wie vorstehend beschrieben, liegt der Vorteil der Aufteilung in ein Unter- und oder Teilvolumen darin, dass in einem Teilbereich die Verteilung des Erwartungswertes der Energiedeposition auch bei Ionenstrahlen nahezu gleichförmig vorliegt, und damit der Verteilung bei Photonenstrahlen ähnelt.

Deshalb sollte der biologische Effekt in diesen kleinen Volumina demjenigen entsprechen, den man für Photonenstrahlung bei gleich großer Dosis erwartet. Somit ist es dadurch ermöglicht, die biologische Wirkung von Ionenstrahlen aus derjenigen von Photonenstrahlen herzuleiten.

Im Verfahrenschritt 280 wird eine Photonendosis PD1 ermittelt, die zum Erreichen derselben Ausbeute korrelierter Schäden erforderlich gewesen wäre. Die korrelierten Schäden sind hierbei typischerweise Kombinationen von Doppelstrangbrüchen, die innerhalb eines vorgegebenen Abstandes induziert werden. Als korrelierte Schäden können aber auch Kombinationen von Doppelstrangbrüchen mit Einzelstrangbrüchen oder anderen, für die Zellfunktionen relevanten Schädigungen der DNA betrachtet werden.

Im Verfahrenschritt 290 wird aus einer zweiten Photonendosiseffektkurve PEK2 für den beobachtbaren Effekt ein zu der Photonendosis PD1 gehöriger Effekt E1 bestimmt. Bei der Bestrahlung von Tumorgewebe kann der beobachtbare Effekt zum Beispiel die Tumorzerstörung sein. Der beobachtbare Effekt kann aber auch das umliegende gesunde Normalgewebe betreffen; zum Beispiel kann es sich um die Schädigung der Haut handeln, die im Eingangskanal des Bestrahlungsfeldes vor dem Tumor liegt. Die Photonendosiseffektkurve PEK2 würde in diesen Fällen eine Abhängigkeit der Tumorzerstörung bzw. der Hautschädigung als Funktion der Bestrahlungsdosis repräsentieren.

Im Verfahrenschritt 300 wird ein beobachtbarer Effekt E2 für die gegebene Fluenzverteilung aus der Skalierung des beobachtbaren Effekts E1 entsprechend dem Verhältnis aus der Photonendosis PD1 und der im sensiblen Volumen entsprechend der Fluenzverteilung deponierten Ionendosis bestimmt. Bevorzugt wird im Verfahrenschritt 300 ein beobachtbarer Effekt E2 für die gegebene Fluenzverteilung aus der Skalierung des beobachtbaren Effekts E1 entsprechend dem Verhältnis aus der Anzahl korrelierter Schäden nach Ionenbestrahlung ($AkS_2$) zur Anzahl korrelierter Schäden nach Photonenbestrahlung ($AkS_1$) bestimmt.

Im Verfahrenschritt 310 wird die effektive Dosis als diejenige Photonendosis PD2 bestimmt, die zu demselben Effekt E2 führen würde wie die Ionendosis ID. Die relative biologische Wirksamkeit ergibt sich dann aus dem Verhältnis der Photonendosis PD2 zur Ionendosis ID.

Mit dem Verfahren 200 lassen sich Vorhersagen über beobachtbare Effekte, beispielsweise die Überlebenswahrscheinlichkeit von Zellen in einer bestrahlten Zellkultur, die Wahrscheinlichkeit der Tumorzerstörung oder die Wahrscheinlichkeit von Normalgewebsschädigungen nach Bestrahlung mit einem Partikelstrahl 34a machen.

Typischerweise wird mit dem Verfahren 200 die Wirkung über den gesamten, für den Einsatz in der Tumortherapie relevanten Energiebereich als auch über einen weiten Bereich verschiedener Ionensorten korrekt beschrieben. Beispielrechnungen, die mit dem Verfahren 200 durchgeführt wurden, sind in den nachfolgenden Figuren dargestellt und beschrieben.

Dabei kann den Berechnungen die folgenden Beschreibungen und Parameter zugrunde gelegt werden, wobei diese nicht zwingend für die vorgeschlagene Bestimmung der effektiven Dosisverteilung sind:

1) Der Zellkern als sensitives Volumen wird als Zylinder mit einem Volumen von $500 \mu m^3$ simuliert. Der Zellkernradius wird mittels experimenteller Daten bestimmt. Die Zellkernhöhe ergibt sich entsprechend.

2) Die radiale Dosisverteilung um einzelne Ionenspuren ergibt sich gemäß Gleichung (11). Dabei ist $r_{min}$=v/c*6.5 nm energieabhängig, wobei v die Ionen- und c die Lichtgeschwindigkeit sind.

3) PEK1 wird aus experimentellen Daten zu $N_{DSB}=\gamma_{DSB}*D_{\gamma}$ mit $\gamma_{DSB}$=30 DSBs/Gy/Zelle und $D_{\gamma}$ ist die Photonendosis. Zusätzlich wird ein weiterer Clustereffekt von Einzelstrangbrüchen (ESB) wie in (Elsässer und Scholz 2007, Radiation Research Vol. 167, 319-329) berücksichtigt.

4) PEK2 ergibt sich aus:

$$S(D) = \begin{cases} e^{-\alpha D - \beta D^2} & : \quad D \leq D_t \\ S_t \, e^{-s \cdot [\eta(D)D - D_t]} & : \quad D > D_t \end{cases} \quad (11)$$

wobei $\alpha$, $\beta$ die linear-quadratischen Parameter sind, die in der Regel durch "invitro" -Messungen oder klinische Daten gegeben sind. $s=\alpha+2\beta D_t$ ist die Steigung oberhalb eines Schwellwertes $D_t$, oberhalb dessen nach experimenteller Kenntnis die PEK2 in einen rein exponentiellen Verlauf übergeht. $S_t$ ist die Steigung für $D>D_t$ und $\eta$ quantifiziert den Clustereffekt von ESB (siehe oben und Elsässer und Scholz 2007, Radiation Research, Vol. 167, 319-329).

5) Korrelierte Schäden werden in den Beispielrechnungen als DSB-Paare mit einem Abstand kleiner als 440 nm angesehen. Dieser Wert wurde unter Anwendung des erfindungsgemäßen Verfahrens anhand experimenteller Daten optimiert. Er ist unabhängig von Ionensorte, Ionenenergie, Dosis oder biologischem Material.

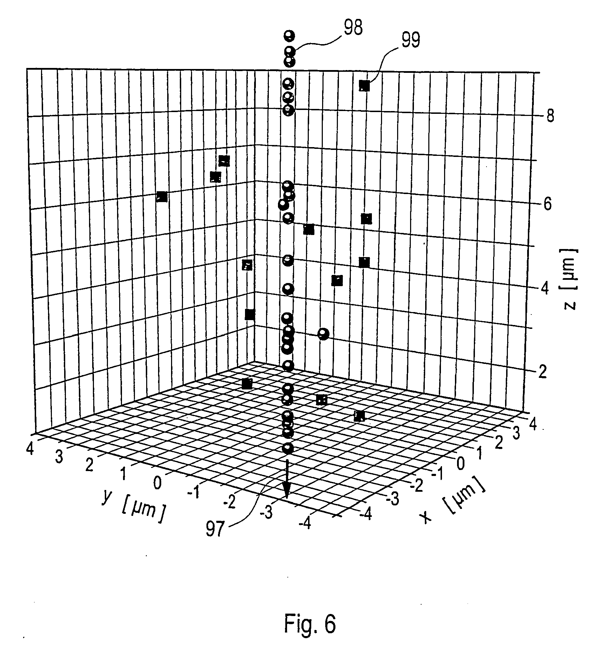

Fig. 6 zeigt eine berechnete Verteilungen von Doppelstrangbrüchen nach Bestrahlung eines Zellkerns mit einem Radius von 5 $\mu$m mit einem Ion (11 MeV/u, 153.5 keV/$\mu$m) bzw. Photonen. Dabei wurde für beide Strahlenarten die gleiche Gesamt-Energiedeposition $D_1$=0.3 Gy zugrunde gelegt, die der Dosisdeposition des einzelnen Ions im Zellkern entspricht. Die drei Achsen x, y, z stellen jeweils eine Längenausdehnung des Zellkerns in x-Richtung, y-Richtung und z-Richtung dar, wobei die Zahlenangabe einer Länge in Mikrometern entspricht. Die kreisförmigen Symbole 98 stellen jeweils einen Doppelstrangbruch nach Durchquerung eines Ions durch den Zellkern dar, wogegen die quadratischen Symbole 99 jeweils einen Doppelstrangbruch nach Bestrahlung mit Photonen darstellen. Die Flugbahn oder Trajektorie des Ions als solche ist mit dem Pfeil 97 gekennzeichnet. Es ist deutlich erkennbar, dass die Doppelstrangbrüche nach Ionenbestrahlung in einem engen Bereich um die Ionenbahn herum lokalisiert sind. Die Doppelstrangbrüche nach Photonenbestrahlung, die mit dem Symbol 99 gekennzeichnet sind, sind dagegen stochastisch und annähernd gleichförmig innerhalb des gesamten Zellkernvolumens verteilt.

Aufgrund der unterschiedlichen räumlichen Verteilung ist es für Ionenstrahlen deutlich wahrscheinlicher, korrelierte Schäden, d.h. zum Beispiel zwei Doppelstrangbrüche innerhalb eines vorgegebenen Abstandes von beispielsweise 440 nm zu induzieren.

Als Maß für die Wahrscheinlichkeit der Induzierung oder alternativ der Ausbeute korrelierter Schäden kann das Verhältnis der Anzahl korrelierter Schäden, beispielsweise Doppelstrangbruch-Paare (DSB-Paare), zur Gesamtzahl der induzierten Einzelschäden oder bevorzugt der isolierten und korrelierten Schäden betrachtet werden. Um mit Photonenstrahlen eine ähnliches Verhältnis der Anzahl korrelierter Schäden AkS zur Gesamtzahl von Einzelschäden oder isolierten und korrelierten Schäden wie mit Ionenstrahlen zu induzieren, ist eine deutlich höhere Dosis notwendig.

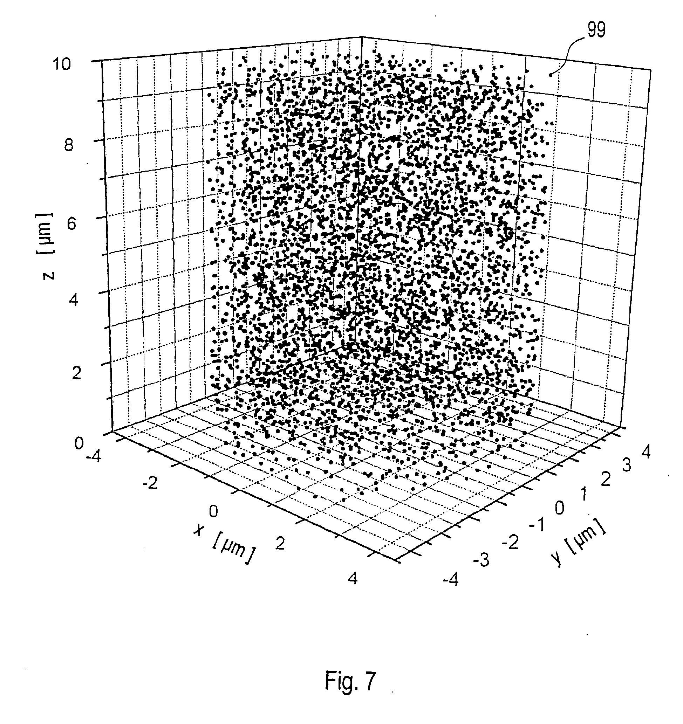

Fig. 7 zeigt die berechnete Verteilung von Doppelstrangbrüchen 99 nach Photonenbestrahlung bei einer Dosis, die zum gleichen Verhältnis korrelierter Schäden zu Gesamtschäden führt wie die in Fig. 6 gezeigte Verteilung für Ionenstrahlen. Für ein geeignet gewähltes Untervolumen des Zellkerns kann deshalb die in Fig. 7 gezeigte Verteilung als repräsentativ für die Wirkung eines in Fig. 6 gezeigten Ionenstrahls angesehen werden und damit die Wirkung eines Ionenstrahls aus der Wirkung von konventionellen Photonenstrahlen hergeleitet werden. Das auf diesem Verfahren beruhende Modell wird als generalisiertes Local-Effect-Model (GLEM) bezeichnet.

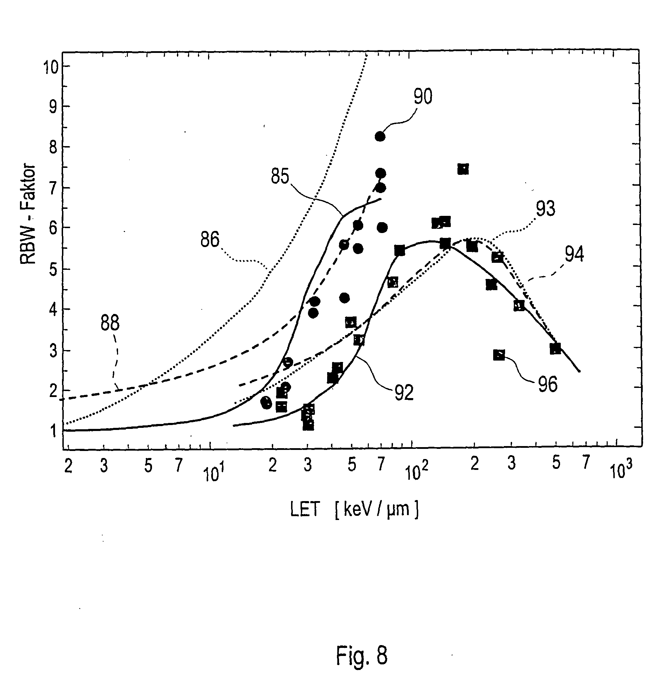

In Fig. 8 zeigt eine mit Hilfe des GLEM durchgeführte Berechnung der relativen biologischen Wirksamkeit für die Inaktivierung von HSG (Human Salivary Gland)-Zellen nach Bestrahlung mit Helium- und Kohlenstoff-Ionen. Die relative biologische Wirksamkeit (RBW) ist auf der y-Achse als Funktion des linearen Energietransfers (LET) auf der x-Achse aufgetragen. Die der Berechnung zugrunde liegenden Photonendaten für PEK2 ($\alpha$=0.313 Gy$^{-1}$, $\beta$=0.0615 Gy$^{-2}$) wurden (Furusawa et al. 2000 Radiation Research, Vol. 154, S. 485-496) entnommen, $D_t$=18 Gy und der Zellkernradius ist 5 $\mu$m.

Die Kurven 85 und 92 bezeichnen eine gemäß des Verfahrens GLEM berechnete funktionelle Beziehung zwischen dem maximalen RBW-Faktor RBW$_\alpha$=$\alpha_{ion}$/$\alpha_x$ und dem LET für eine Bestrahlung mit Helium- und Kohlenstoffionen sowie einen Vergleich mit experimentellen Daten 90 und 96 (exp. Daten entnommen aus Furusawa et al. 2000 Radiation Research, Vol. 154, S. 485-496). Kurve 85 ist eine gemäß GLEM, Kurve 86 gemäß LEM III und Kurve 88 ist eine gemäß LEM II berechnete Kurve für Heliumionen. Die Messdaten, die durch runde Symbole bezeichnet und stellvertretend durch 90 bezeichnet sind, sind diejenigen, die nach einer Bestrahlung eines Zielvolumens 44 mit Heliumionen ermittelt wurden. Analog zeigt Kurve 92 eine Berechnung mit GLEM, Kurve 93 mit LEM III und Kurve 94 zeigt eine Berechnung mit LEM II für Kohlenstoffionen; und die quadratischen Symbole, stellvertretend symbolisiert durch 96, zeigen die zugehörigen experimentellen Werte. Es ist deutlich erkennbar, dass die Kurve 85 und die Kurve 92 die experimentellen Werte am besten widerspiegeln. Insbesondere für einen niedrigen LET ist deutlich erkennbar, dass die mit GLEM berechneten Kurven die Lage der Messwerte 90 und 96 besser vorhersagen.

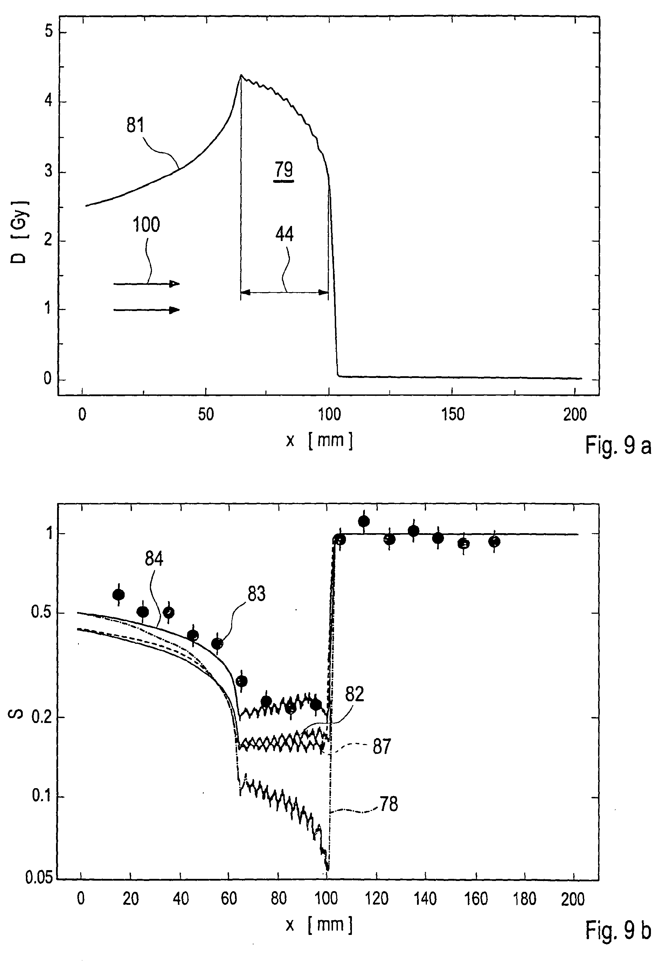

Dies wird durch den in Fig. 9 gezeigten Vergleich erhärtet. Fig.9a zeigt ein physikalisches Dosisprofil 81, für ein mit Energie-modulierten Heliumionen (4cm extended Bragg-Peak in einer wasseräquivalenten Tiefe von 8cm) bestrahlten Zielvolumens 44 in Abhängigkeit von der Eindringtiefe x in mm. Mit der Bezugsziffer 79 ist die im Zielvolumen 44 deponierte Dosis D bezeichnet. Die Pfeile 100 bezeichnen den Ionenstrahl. Als Vergleich mit experimentellen Daten ist das experimentell ermittelte Überleben von CHO-Zellen mit den Parametern der PEK2 $\alpha$=0.228 Gy$^{-1}$, $\beta$=0.02 Gy$^{-2}$ und $D_t$=35 Gy (Zellkernradius 5$\mu$m) mit dem berechneten Überleben verglichen.

In Fig. 9b stellt Kurve 87 ein Ergebnis der Berechnung gemäß LEM I, Kurve 82 gemäß der Berechnung gemäß LEM II, Kurve 78 gemäß der Berechnung mit LEM III, und Kurve 84 gemäß der Berechnung gemäß des GLEM dar. Die gefüllten Kreise, stellvertretend markiert durch 83, repräsentieren die experimentellen Ergebnisse (Veröffentlichung Müller, GSI Report 2004). Auch hier zeigt die Berechnung mit GLEM eine wesentlich bessere Übereinstimmung mit den experimentellen Daten im Vergleich zu den Modellen LEM I - LEM III.

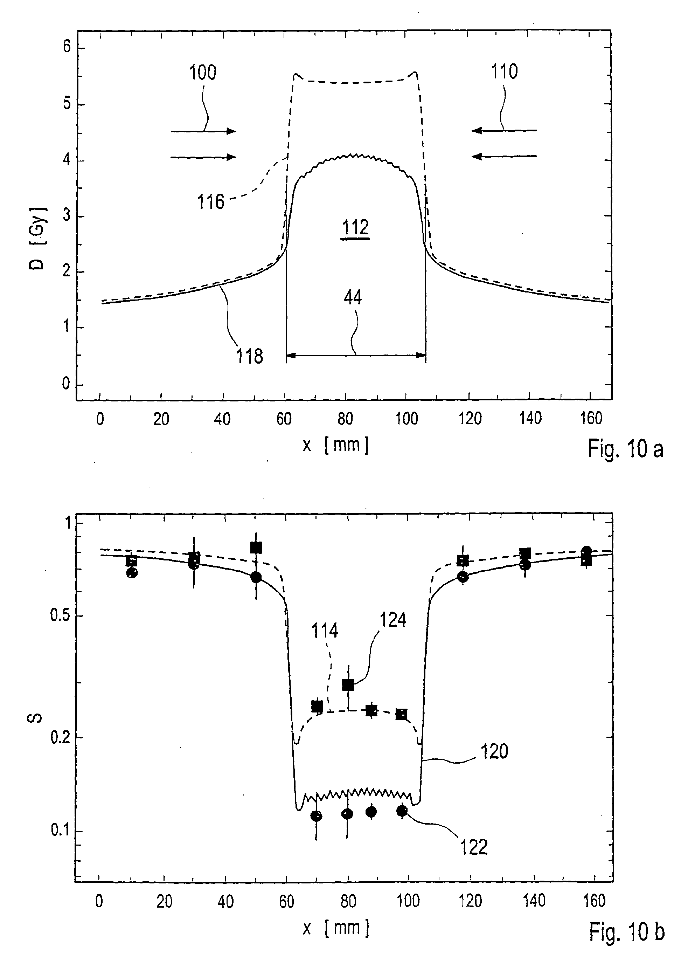

Fig. 10 zeigt in einem weiteren Vergleich die Berechnung der Wirkung von Protonen- und Kohlenstoffstrahlen in einem therapie-ähnlichen Bestrahlungsfeld zusammen mit den entsprechenden experimentellen Daten für CHO-Zellen mit den ebenfalls experimentell bestimmten PEK2-Parametern $\alpha$=0.105 Gy$^{-1}$, $\beta$=0.025 Gy$^{-2}$ und $D_t$=40 Gy (Zellkernradius 6 $\mu$m).

Fig.10 a zeigt die physikalische Dosisverteilung mit zwei opponierenden Feldern, die unter einem Winkel von 180 Grad eingestrahlt wurden. Die Bestrahlung des Zielvolumens 44 mit dem Partikelstrahl 34a ist hierbei aus einer ersten Richtung, die durch die Pfeile 100 schematisch gezeigt ist, und einer zweiten Richtung, die durch die Pfeile 110 schematisch gezeigt ist, erfolgt. Kurve 116 zeigt ein physikalisches Dosisprofil D der Kohlenstoffbestrahlung und Kurve 118 ein physikalisches Dosisprofil D der Protonenbestrahlung. Die Dosis ist mit D bezeichnet und auf der y-Achse aufgetragen und die Längeneinheit x auf der x-Achse. Die Lage des Zielvolumens 44 ist durch den Bereich 112 schematisch dargestellt.

Fig. 10b zeigt das berechnete und experimentell bestimmte Überleben der CHO-Zellen. Die quadratischen Symbole 124 sind das gemessene Überleben und die Kurve 114 das berechnete Überleben nach Protonenbestrahlung. Die runden Symbole 122 stellen das gemessene Überleben und die Kurve 120 das berechnete Überleben nach Kohlenstoffbestrahlung dar. Aus dem Vergleich der Kurve 114 mit den Messergebnissen 124 für eine erfolgte Protonenstrahlung und dem Vergleich der Kurve 120 mit den Messdaten 122 ist ersichtlich, dass die effektive Dosisverteilung, die mit dem Modell GLEM mit dem vorgeschlagenen Verfahren 200 berechnet wurde, die experimentellen Daten sehr gut wiedergibt.

Die Vergleiche in den Fig. 8-10 zeigen somit, dass die Berechnungen mittels des erfindungsgemäßen Modells GLEM am zuverlässigsten zur Vorhersage der effektiven Dosisverteilung insbesondere für die Partikeltherapie geeignet sind.

In dem gezeigten Verfahren zur Bestrahlung und/oder zur Bestrahlungsplanung ist ein Verfahren zur Bestimmung einer Wirkung eines Partikelstrahls 34a in einem zumindest teilweise bestrahlten oder zu bestrahlenden Material enthalten. Bei diesem Verfahren wird zumindest aus einem Parameter, der den Partikelstrahl 34a charakterisiert, und aus zumindest einer Eigenschaft des Materials, die Wirkung des Partikelstrahls in dem Material zumindest teilweise auf Basis einer mikroskopischen Schadenskorrelation bestimmt. Das Verfahren zur Bestrahlung und/oder zur Bestrahlungsplanung ist ein Verfahren zur Bestimmung einer Wirkung eines Partikelstrahls 34a in einem zumindest teilweise bestrahlten oder zu bestrahlenden Material und kann ferner die im folgenden aufgeführten Schritte enthalten:

- Ermittlung der mikroskopische Schadenskorrelation unter Verwendung einer räumlichen mikroskopischen Schadensverteilung;

- Ermitteln der räumlichen mikroskopischen Schadensverteilung zumindest teilweise aus einer mikroskopischen Dosisverteilung, wobei diese durch den Partikelstrahl 34a erzeugt wird, wobei die Wahrscheinlichkeit einer lokalen Schadensinduktion zumindest teilweise aus einer ersten Photonendosiseffektkurve abgeleitet wird;

- Bestimmen eines Erwartungswert für eine Anzahl korrelierter Schäden in einem geeignet gewählten Teilvolumen eines sensitiven Volumens zumindest teilweise aus der räumlichen, mikroskopischen Schadensverteilung bestimmt wird;

- Ermitteln einer Photonendosis, die zum Erreichen annähernd derselben Ausbeute korrelierter Schäden entsprechend dem für den Partikelstrahl erwarteten Wert der Anzahl korrelierter Schäden erforderlich gewesen wäre;

- Bestimmen eines zu der Photonendosis gehörigen Effektes oder einer zugehörigen Wirkung zumindest teilweise aus einer zweiten Photonendosiseffektkurve;

- Hinterlegen von zu den verschiedenen Parametern des Partikelstrahls und für die verschiedenen Parameter ermittelten jeweiligen Wirkungen in einer Speichereinheit, wobei die für verschiedene Parameter, die den Partikelstrahl 34a charakterisieren und/oder zumindest eine Eigenschaft des Materials, insbesondere verschiedene Eigenschaften des Materials jeweils die Wirkung des Partikelstrahls in dem zu bestrahlenden Material auf Basis der mikroskopischen Schadenskorrelation bestimmt wird.

Somit ist die Wirkung eines Partikelstrahls 34a in einem zumindest teilweise bestrahlten oder zu bestrahlenden Material in einem separaten Verfahren bestimmbar und in Form von Datensätzen, Tabellen, Wertetabellen, etc. getrennt in einem Speichermedium speicherbar. Diese Datensätze, Tabellen etc. können einem bekannten und von dem vorstehend genannten Verfahren verschiedenen Verfahren zur Bestrahlung, insbesondere zur Bestrahlungsplanung als Eingabedaten eingegeben werden.

## Patentansprüche

1. Verfahren zur Bestimmung einer Wirkung eines Partikelstrahls (34a) in einem zumindest teilweise bestrahlten oder zu bestrahlenden Material, welches zumindest teilweise ein biologisches Material aufweist, wobei aus zumindest einem Parameter, der den Partikelstrahl (34a) charakterisiert, und aus zumindest einem Parameter, der eine Materialeigenschaft charakterisiert, die Wirkung des Partikelstrahls in dem Material zumindest teilweise auf Basis einer mikroskopischen Schadenskorrelation von Doppelstrangbrüchen einer DNS mit Abständen von ≥100 nm bestimmt wird.

2. Verfahren gemäß Anspruch 1, wobei die mikroskopische Schadenskorrelation im Submikrometerbereich erfolgt.

3. Verfahren gemäß Anspruch 1 oder 2, wobei die mikroskopische Schadenskorrelation unter Verwendung einer räumlichen mikroskopischen Schadensverteilung ermittelt wird.

4. Verfahren gemäß Anspruch 3, wobei die räumliche mikroskopische Schadensverteilung zumindest teilweise aus einer mikroskopischen Dosisverteilung ermittelt wird, die durch den Partikelstrahl (34a) erzeugt wird, wobei die Wahrscheinlichkeit einer lokalen Schadensinduzierung zumindest teilweise aus einer ersten Photonendosiseffektkurve abgeleitet wird.

5. Verfahren gemäß einem der vorhergehenden Ansprüche, wobei bei der Bestimmung der mikroskopischen Schadenskorrelation ein Erwartungswert für eine Anzahl korrelierter Schäden in einem geeignet gewählten Teilvolumen eines sensitiven Volumens zumindest teilweise aus der räumlichen, mikroskopischen Schadensverteilung bestimmt wird.

6. Verfahren gemäß einem der vorhergehenden Ansprüche, wobei eine Photonendosis ermittelt wird, die zum Erreichen annähernd derselben Ausbeute korrelierter Schäden entsprechend dem für den Partikelstrahl erwarteten Wert der Anzahl korrelierter Schäden erforderlich gewesen wäre.

7. Verfahren zur Bestrahlungsplanung für ein Zielvolumen in einem Bestrahlungsvolumen mittels eines Partikelstrahls (34a), umfassend folgende Schritte:

- Vorgeben eines Zielvolumens (44) in einem Bestrahlungsvolumen (46),
- Ermitteln einer Fluenz- und/oder Energieverteilung innerhalb des, das Zielvolumen (44) ausweisenden Bestrahlungsvolumens (46);
- Ermitteln einer aus der Fluenz- und/oder Energieverteilung resultierenden effektiven Dosisverteilung, wobei die Wirkung des Partikelstrahls im Material des Bestrahlungsvolumens (46) verwendet wird, welche nach einem Verfahren gemäß einem der Ansprüche 1 bis 6 ermittelt worden ist.

8.  Verfahren gemäß Anspruch 7, wobei das Verfahren zur oder bei der Erstellung eines Bestrahlungsplans für das Zielvolumen (44) und/oder bei der Validierung eines Bestrahlungsplanes für das Zielvolumen (44) verwendet wird.

9.  Verfahren gemäß einem der vorhergehenden Ansprüche 7 oder 8, wobei ein beobachtbarer Effekt für die gegebene Fluenzverteilung zumindest teilweise aus einer Skalierung eines beobachtbaren Effekts für eine Photonendosis entsprechend dem Verhältnis der Photonendosis und einer im sensitiven Volumen entsprechend der Fluenzverteilung deponierten Dosis bestimmt wird.

10. Verfahren gemäß einem der vorhergehenden Ansprüche 7 bis 9, wobei die effektive Dosisverteilung zumindest teilweise aus der Wirkung, insbesondere dem beobachtbaren Effekt der zugehörigen Photonendosis bestimmt wird.

11. Verfahren gemäß einem der vorhergehenden Ansprüche, wobei das sensitive Volumen zumindest teilweise mindestens ein Unter- und/oder Teilvolumen des biologischen Materials, insbesondere eine Zelle, aufweist.

12. Bestrahlungsplanungseinrichtung mit einer Rechnereinheit, die derart ausgebildet und eingerichtet ist, dass mit ersterer ein Verfahren nach einem der Ansprüche 1 bis 11 durchführbar ist.

13. Bestrahlungsvorrichtung, die eine Bestrahlungsplanungseinrichtung nach Anspruch 12 aufweist.

14. Bestrahlungsvorrichtung aufweisend eine Strahlmodifikationseinrichtung, die derart ausgebildet und eingerichtet ist, dass sie während der Bestrahlung eines Zielvolumens in einem Bestrahlungsvolumen mittels eines Partikelstrahls (34a) ein Verfahren durchführt, welches folgende Schritte umfasst:

- Ermitteln einer Fluenz- und/oder Energieverteilung innerhalb des, das Zielvolumen (44) aufweisenden Bestrahlungsvolumens (46);
- Ermitteln einer aus der Fluenz- und/oder Energieverteilung resultierenden effektiven Dosisverteilung, wobei die Wirkung des Partikelstrahls im Material des Bestrahlungsvolumens (46) verwendet wird, welche zumindest teilweise auf der Basis der mikroskopischen Schadenskorrelation nach einem Verfahren gemäß einem der Ansprüche 1 bis 6 ermittelt worden ist;
- Applikation der derart berechneten effektiven Dosisverteilung in einem Zielvolumen.

## Claims

1.  Method for determining an effect of a particle beam (34a) in a material at least partially irradiated or to be irradiated and which at least in part incorporates a biological material, wherein from at least one parameter characterizing the particle beam (34a) and from at least one parameter characterizing a material property, the effect of the particle beam in the material is determined at least partially on the basis of a microscopic damage correlation of double strand breaks of a DNA with intervals of $\geq 100$ nm.

2.  Method according to claim 1, wherein the microscopic damage correlation is in the sub-micrometer range.

3.  Method according to claim 1 or 2, wherein the microscopic damage correlation is determined using a spatial microscopic damage distribution.

4.  Method according to claim 3, wherein the spatial microscopic damage distribution is determined at least partially from a microscopic dose distribution generated by the particle beam (34a), wherein the probability of a local damage being induced is derived at least partially from a first photon dose effect curve.

5.  Method according to one of the preceding claims, wherein during determination of the microscopic damage correlation an expected value for a number of correlated damage events in a suitably selected partial volume of a sensitive volume is determined at least partially from the spatial microscopic damage distribution.

6. Method according to one of the preceding claims, wherein a photon dose is determined which would have been required to achieve approximately the same yield of correlated damage events corresponding to the value of the number of correlated damage events expected for the particle beam.

7. Method for irradiation planning for a target volume in an irradiation volume by means of a particle beam (34a), comprising the following steps:

    - specifying a target volume (44) in an irradiation volume (46),
    - determining a fluence and/or energy distribution inside the irradiation volume (46) incorporating the target volume (44);
    - determining an effective dose distribution resulting from the fluence and/or energy distribution, wherein the effect of the particle beam in the material of the irradiation volume (46) is used which has been determined using a method according to one of claims 1 to 6.

8. Method according to claim 7, wherein the method is used for or during the drafting of an irradiation plan for the target volume (44) and/or during the validation of an irradiation plan for the target volume (44).

9. Method according to one of the preceding claims 7 or 8, wherein an observable effect for the given fluence distribution is determined at least partially from a scaling of an observable effect for a photon dose corresponding to the ratio of the photon dose and a dose deposited in the sensitive volume corresponding to the fluence distribution.

10. Method according to one of the preceding claims 7 to 9, wherein the effective dose distribution is determined at least partially from the effect, in particular the observable effect, of the associated photon dose.

11. Method according to one of the preceding claims, wherein the sensitive volume incorporates at least in part at least one sub-volume and/or partial volume of the biological material, in particular a cell.

12. Irradiation planning device with a computing unit that is designed and configured such that with the former a method according to one of claims 1 to 11 can be implemented.

13. Irradiation device incorporating an irradiation planning device according to claim 12.

14. Irradiation device incorporating a beam modification device that is designed and configured such that during irradiation of a target volume in an irradiation volume by means of a particle beam (34a) it performs a method comprising the following steps:

    - determining a fluence and/or energy distribution inside the irradiation volume (46) incorporating the target volume (44);
    - determining an effective dose distribution resulting from the fluence and/or energy distribution, wherein the effect of the particle beam in the material of the irradiation volume (46) is used which has been determined at least partially on the basis of a microscopic damage correlation using a method according to one of claims 1 to 6;
    - application of the effective dose distribution thus computed in a target volume.

**Revendications**

1. Procédé de détermination d'un effet d'un rayonnement corpusculaire (34a) dans un matériau au moins partiellement irradié ou à irradier, lequel présente au moins partiellement un matériau biologique, sachant qu'au moins à partir d'un paramètre qui caractérise le rayonnement corpusculaire (34a) et au moins à partir d'un paramètre qui caractérise une propriété du matériau, l'effet du rayonnement corpusculaire dans le matériau est déterminé au moins partiellement sur la base d'une corrélation microscopique de dommages sous forme de cassures du double brin d'un DNA avec intervalles $\geq$ 100 nm.

2. Procédé selon la revendication 1, dans lequel la corrélation microscopique de dommages a lieu à l'échelle submicrométrique.

3. Procédé selon la revendication 1 ou 2, dans lequel la corrélation microscopique de dommages est déterminée en utilisant une répartition microscopique de dommages dans l'espace.

4. Procédé selon la revendication 3, dans lequel la répartition microscopique de dommages dans l'espace est déterminée au moins partiellement à partir d'une répartition microscopique de doses générée par le rayonnement corpusculaire (34a), sachant que la probabilité d'une induction locale de dommages est déduite au moins partiellement à partir d'une première courbe dose-effet des photons.

5. Procédé selon l'une des revendications précédentes, dans lequel lors de la détermination de la corrélation microscopique de dommages, une valeur escomptée pour un nombre de dommages corrélés dans volume partiel choisi de manière appropriée d'un volume sensitif est déterminée au moins partiellement à partir de la répartition microscopique de dommages dans l'espace.

6. Procédé selon l'une des revendications précédentes, dans lequel est déterminée une dose de photons qui aurait été nécessaire pour atteindre approximativement le même taux de dommages corrélés correspondant à la valeur escomptée de dommages corrélés pour le rayonnement corpusculaire.

7. Procédé pour un programme d'irradiation pour un volume cible dans un volume d'irradiation au moyen d'un rayonnement corpusculaire (34a), comprenant les étapes suivantes :

    - définition d'un volume cible (44) dans un volume d'irradiation (46) ;
    - détermination d'une répartition de fluence et/ou d'énergie au sein du volume d'irradiation (46) présentant le volume cible (44) ;
    - détermination d'une répartition effective de doses résultant de la répartition de fluence et/ou d'énergie, sachant que l'effet du rayonnement corpusculaire utilisé dans le matériau du volume d'irradiation (46) est celui qui a été déterminé d'après un procédé selon l'une des revendications 1 à 6.

8. Procédé selon la revendication 7, dans lequel est mis en oeuvre le procédé pour ou lors de l'établissement d'un plan d'irradiation pour le volume cible (44) et/ou lors de la validation d'un plan d'irradiation pour le volume cible (44).

9. Procédé selon l'une des revendications 7 ou 8 précédentes, dans lequel un effet observable pour la répartition de fluence donnée est déterminé au moins partiellement à partir d'une graduation d'un effet observable pour une dose de photons correspondant au rapport entre la dose de photons et une dose déposée dans le volume sensitif et correspondant à la répartition de fluence.

10. Procédé selon l'une des revendications 7 à 9 précédentes, dans lequel la répartition effective de doses est déterminée au moins partiellement à partir de l'effet, en particulier de l'effet observable, de la dose de photons associée.

11. Procédé selon l'une des revendications précédentes, dans lequel le volume sensitif présente au moins partiellement au moins un sous-volume et/ou un volume partiel du matériau biologique, en particulier une cellule.

12. Dispositif de planification d'irradiation comprenant un ordinateur conçu et aménagé de manière telle qu'un procédé selon l'une des revendications 1 à 11 peut être réalisé avec ledit dispositif.

13. Dispositif d'irradiation présentant un dispositif de planification d'irradiation selon la revendication 12.

14. Dispositif d'irradiation présentant un dispositif de modification de rayonnement et qui est conçu et aménagé de telle manière que, pendant l'irradiation d'un volume cible dans un volume d'irradiation au moyen d'un rayonnement corpusculaire (34a), il réalise un procédé comprenant les étapes suivantes :

    - détermination d'une répartition de fluence et/ou d'énergie au sein du volume d'irradiation (46) présentant le volume cible (44) ;
    - détermination d'une répartition effective de doses résultant de la répartition de fluence et/ou d'énergie, sachant qu'est utilisé l'effet du rayonnement corpusculaire dans le matériau du volume d'irradiation (46), lequel effet ayant été déterminé au moins partiellement sur la base de la corrélation de dommages d'après un procédé selon l'une des revendications 1 à 6 ;
    - application dans un volume cible de la répartition effective de doses calculée de cette manière.

Fig. 1

Fig. 2 a

Fig. 2 b

EP 2 670 484 B1

Photonen

74

Fig. 3 d

Kohlenstoff lokal

80

Fig. 3 c

x [µm]

x [nm]

D [Gy]

D [Gy]

x 10000

76

Kohlenstoff-Ionen

78

72

72

72

72

Fig. 3 a

x [µm]

y [µm]

D [Gy]

Fig. 3 b

200

210

220

230

nein — 235

ja

240

Fig. 4

230

250

260

270

280

290

300

310

Fig. 5

Fig. 6

Fig. 7

Fig. 8

Fig. 9 a

Fig. 9 b

Fig. 10 a

Fig. 10 b

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **KRÄMER ; SCHOLZ.** *Physics in Medicine and Biology,* 2000, vol. 45, 3319-3330
- **SCHOLZ et al.** *Radiation Environmental Biophysics,* 1997, vol. 36, 59-66
- **KRÄMER ; SCHOLZ.** *Physics in Medicine and Biology,* 2006, vol. 51, 1959-1970
- **ELSÄSSER ; SCHOLZ.** *Radiation Research,* 2007, vol. 167, 319-329
- **ELSÄSSER et al.** *International Journal of Radiation Oncology Biology Physics,* 2008, vol. 71, 866-872
- **FURUSAWA et al.** *Radiation Research,* 2000, vol. 154, 485-496
- **MÜLLER.** *GSI Report,* 2004